(19) Europäisches Patentamt
European Patent Office
Office européen des brevets

(11) EP 1 223 210 B1

(12) **EUROPÄISCHE PATENTSCHRIFT**

(45) Veröffentlichungstag und Bekanntmachung des Hinweises auf die Patenterteilung:
**03.03.2004 Patentblatt 2004/10**

(51) Int Cl.[7]: **C09K 19/32**, C09K 19/42, C07C 25/00

(21) Anmeldenummer: **02000193.9**

(22) Anmeldetag: **10.01.2002**

(54) **Mehrfach fluorierte Fluorene und ihre Verwendung in Flüssigkristallmischungen**

Polyfluorinated fluorenes and use thereof in liquid crystal mixtures

Fluorènes polyfluorés et leur utilisation dans des compositions liquides cristallines

(84) Benannte Vertragsstaaten:
**DE FR GB**

(30) Priorität: **11.01.2001 DE 10101021**

(43) Veröffentlichungstag der Anmeldung:
**17.07.2002 Patentblatt 2002/29**

(73) Patentinhaber: **CLARIANT INTERNATIONAL LTD.**
**4132 Muttenz (CH)**

(72) Erfinder:
- **Schmidt, Wolfgang Dr.**
  **63303 Dreieich (DE)**
- **Wingen, Rainer Dr.**
  **65795 Hattersheim (DE)**
- **Hornung, Barbara Dl.**
  **63594 Hasselroth (DE)**

(74) Vertreter: **Isenbruck, Günter, Dr. et al**
**Isenbruck, Bösl, Hörschler, Wichmann, Huhn, Patentanwälte**
**Theodor-Heuss-Anlage 12**
**68165 Mannheim (DE)**

(56) Entgegenhaltungen:
- **DATABASE CA [Online] CHEMICAL ABSTRACTS SERVICE, COLUMBUS, OHIO, US; SHUNDO, RYUJI ET AL: "Fluorene derivative compound and liquid crystal composition containing it" retrieved from STN Database accession no. 133:66049 XP002196880 & JP 2000 178211 A (CHISSO CORP., JAPAN) 27. Juni 2000 (2000-06-27)**
- **DATABASE CA [Online] CHEMICAL ABSTRACTS SERVICE, COLUMBUS, OHIO, US; YANO, HITOSHI ET AL: "Liquid-crystal compound with high negative dielectric anisotropy, its composition, and display device using it" retrieved from STN Database accession no. 130:304122 XP002196881 & JP 10 236992 A (CHISSO CORP., JAPAN) 8. September 1998 (1998-09-08)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Viele Hersteller haben elektrooptische Anzeigen auf der Basis nematischer Flüssigkristalle entwickelt, die seit einigen Jahren beispielsweise im Bereich von Notebook PCs, Personal Digital Assistants und Desktop Monitoren im Einsatz sind. Dabei werden die Technologien TN (Twisted Nematics), STN (Supertwisted Nematics), AM-TN (Active Matrix - Twisted Nematics), AM-IPS (Active Matrix - In Plane Switching), AM-MVA (Active Matrix - Multidomain Vertically Aligned) verwendet, die in der Literatur ausführlich beschrieben werden, siehe z. B. T. Tsukuda, TFT/LCD: Liquid Crystal Displays Addressed by Thin-Film Transistors, Gordon and Breach 1996, ISBN 2-919875-01-9 und darin zitierte Literatur; SID Symposium 1997, ISSN-0097-966X, Seiten 7 bis 10, 15 bis 18, 47 bis 51, 213 bis 216, 383 bis 386, 397 bis 404 und darin zitierte Literatur.

Clark und Lagerwall (US 4,367,924) konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN (Twisted Nematic)-Zellen um bis zu einen Faktor 1000 schnellere Schaltzeiten haben (siehe z. B. EP-A-0 032 362). Aufgrund dieser und anderer günstiger Eigenschaften, z. B. der bistabilen Schaltmöglichkeit und des nahezu blickwinkelunabhängigen Kontrasts, sind FLCs grundsätzlich für Anwendungsgebiete wie Computerdisplays und Fernsehgeräte geeignet, wie ein seit Mai 1995 in Japan von Canon vermarkteter Desktop-Monitor zeigt.

Die einzelnen Bildelemente (Pixel) eines LC-Displays sind üblicherweise in einer x,y-Matrix angeordnet, die durch die Anordnung je einer Serie von Elektroden (Leiterbahnen) entlang der Reihen und der Spalten an der Unter- bzw. Oberseite des Displays gebildet wird. Die Kreuzungspunkte der horizontalen (Reihen-) und vertikalen (Spalten-) Elektroden bilden adressierbare Pixel.

Diese Anordnung der Bildpunkte bezeichnet man üblicherweise als eine passive Matrix. Zur Adressierung wurden verschiedene Multiplex-Schemata entwickelt, wie beispielsweise in Displays 1993, Vol. 14, Nr. 2, S. 86-93 und Kontakte 1993 (2), S. 3-14 beschrieben. Die passive Matrixadressierung hat den Vorteil einer einfacheren Herstellung des Displays und damit verbundenen geringen Herstellkosten, jedoch den Nachteil, daß die passive Adressierung immer nur zeilenweise erfolgen kann, was dazu führt, daß die Adressierungszeit des gesamten Bildschirms bei N Zeilen das N-fache der Zeilenadressierungszeit beträgt.

Bei der sogenannten Aktivmatrix-Technologie (AM-LCD) wird üblicherweise ein nichtstrukturiertes Substrat mit einem Aktivmatrix-Substrat kombiniert. An jedem Pixel des Aktivmatrixsubstrates ist ein elektrisch nichtlineares Element, beispielsweise ein Dünnschichttransistor, integriert. Bei dem nichtlinearen Element kann es sich auch um Dioden, Metall-Insulator-Metall u.ä. Elemente handeln, die vorteilhaft mit Dünnschichtverfahren hergestellt werden und in der einschlägigen Literatur beschrieben sind, siehe z. B. T. Tsukuda, TFT/LCD: Liquid Crystal Displays Addressed by Thin-Film Transistors, Gordon and Breach 1996, ISBN 2-919875-01-9 und darin zitierte Literatur.

Aktivmatrix-LCDs werden üblicherweise mit nematischen Flüssigkristallen im TN-(Twisted Nematics), ECB-(Electrically Controlled Birefringence), VA- (Vertically Aligned) oder IPS- (In Plane Switching) Modus betrieben. In jedem Fall wird durch die aktive Matrix an jedem Bildpunkt ein elektrisches Feld individueller Stärke erzeugt, das eine Orientierungsänderung und damit eine Änderung der Doppelbrechung erzeugt, die wiederum im polarisierten Licht sichtbar ist. Ein Nachteil dieser Verfahren ist derzeit noch die mangelnde Videofähigkeit bedingt durch die langen Schaltzeiten nematischer Flüssigkristalle.

Unter anderem aus diesem Grunde wurden auch Flüssigkristallanzeigen, die auf einer Kombination aus ferroelektrischen Flüssigkristallmaterialien und aktiven MatrixElementen beruhen,vorgeschlagen, siehe z. B. WO 97/12355, Ferroelectrics 1996, 179, 141-152, oder WO 99/60441.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses ist eine einheitliche planare Orientierung der Flüssigkristalle nötig. Eine gute Orientierung in der $S_A$ und $S^*_C$-Phase läßt sich z. B. erreichen, wenn die Phasenfolge der Flüssigkristallmischung mit abnehmender Temperatur lautet:

$$\text{Isotrop} \rightarrow N^* \rightarrow S_A \rightarrow S^*_C$$

Voraussetzung ist, daß der Pitch (Ganghöhe der Helix) in der N*-Phase sehr groß (größer 10 μm) oder, noch besser, völlig kompensiert ist (siehe z. B. T. Matsumoto et al., Proc. of the 6th Int. Display Research Conf., Japan Display, Sept. 30 - Okt. 2, 1986, Tokyo, Japan, S. 468-470; M. Murakami et al., ibid. S. 344-347). Dies erreicht man z. B., indem man zu der chiralen Flüssigkristallmischung, die in der N*-Phase z. B. eine linksdrehende Helix aufweist, einen oder mehrere optisch aktive Dotierstoffe, die eine rechtsdrehende Helix induzieren, in solchen Mengen hinzugibt,

daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C*-Phase wesentlich größer ist als die Dicke des Anzeigeelementes (Mol. Cryst. Liq. Cryst. 1983, 94, 213 und ibid. 1984, 114, 151).

Die optische Schaltzeit $\tau$ [$\mu$s] ferroelektrischer Flüssigkristallsysteme, die möglichst kurz sein soll, hängt von der Rotationsviskosität des Systems $\gamma$ [mPas], der spontanen Polarisation Ps [$nC/cm^2$] und der elektrischen Feldstärke E [V/m] ab nach der Beziehung

$$\tau \sim \frac{\gamma}{Ps \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und/oder eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$ und eine geringe positive oder vorzugsweise negative dielektrische Anisotropie $\Delta\varepsilon$ verlangt (siehe z. B. S. T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll. Zudem ist es notwendig, daß die unterschiedlichen Komponenten in einem breiten Temperaturbereich untereinander gut mischbar sind.

Da aber die Entwicklung von Flüssigkristallmischungen noch in keiner Weise als abgeschlossen betrachtet werden kann, sind die Hersteller von Displays an den unterschiedlichsten Komponenten für Mischungen interessiert.

Insbesondere werden Flüssigkristallmischungen benötigt, die einerseits einen sehr weiten Arbeits-Temperaturbereich aufweisen, andererseits eine möglichst geringe Schwellspannung besitzen, beispielsweise für den Einsatz in Automobilen, in denen ohne weiteres ein Temperaturbereich von -40°C bis 100°C auftreten kann, aber auch für tragbare Geräte wie Mobiltelefone und Notebook-PCs.

Darüber hinaus scheint der Trend hin zu bewegten Bildern mit nematischen Flüssigkristallen nur dann realisierbar zu sein, wenn man den Elektrodenabstand solcher LCDs verringert, denn die Schaltzeiten sind umgekehrt proportional zum Quadrat des Elektrodenabstandes. Dies wiederum zieht unweigerlich eine Verringerung von Helligkeit und Kontrast nach sich, es sei denn, man verwendet Flüssigkristallmischungen mit einem hohen Wert der optischen Anisotropie ($\Delta n$). Gewöhnlich besitzen Materialien mit hoher optischer Anisotropie jedoch auch hohe Viskositäten und wirken daher dem gesetzten Ziel entgegen. Es besteht daher Nachfrage nach neuen, geeigneten Flüssigkristallmischungen und -mischungskomponenten, die einen hohen Klärpunkt und eine hohe optische Anisotropie besitzen, bei gleichzeitig verhältnismäßig geringer Rotationsviskosität.

Aufgabe der vorliegenden Erfindung ist es daher, neue Komponenten für die Verwendung in smektischen oder nematischen Flüssigkristallmischungen bereit zu stellen, die über hohe positive Werte der dielektrischen Anisotropie kombiniert mit einem günstigen Verhältnis von Viskosität und Klärpunkt verfügen. Darüber hinaus sollen die Verbindungen in hohem Maße licht- und UV-stabil sowie thermisch stabil sein. Ferner sollen sie geeignet sein, eine hohe "voltage holding ratio (VHR)" zu realisieren. Außerdem sollten sie synthetisch gut zugänglich und daher potentiell kostengünstig sein.

Für die Verwendung in flüssigkristallinen Mischungen sind Derivate des Fluorens und Fluorenons bereits aus J. Chem. Soc. 1955, 4359, ibid. 1957, 3228, Pramana Suppl. 1975, 1, 397, Bull. Soc. Chim. Fr. 1975, 2066, Mol. Cryst. Liq. Cryst 1985, 127, 341, ibid. 1985, 129, 17, ibid. 1987, 150B, 361, ibid. 1988, 164, 167, ibid. 1990, 182, 339, DE-A 197 20 289, EP-A 0 113 293, EP-B 0 325 035, JP 05 125055, JP 2000 178211 und EP-A 0 455 219 bekannt.

In der JP 10 236992 läßt sich aus der dort angegebenen allgemeinen Formel unter einer Vielzahl anderer Möglichkeiten auch ein 2,7-disubstituiertes 3,4,5,6-Tetrafluorfluorenderivat konstruieren, in der Anmeldung wird jedoch auf die Synthese oder physikalische Eigenschaften entsprechender Verbindungen nicht eingegangen.

Mehrfach fluorierte Fluorenderivate sind beispielsweise aus Can. J. Chem. 1967, 45, 2569-2575, J. Chem. Soc. 1968, 2394, oder Tetrahedron 1969, 25, 565 bekannt. Eine Eignung dieser Moleküle oder deren Derivate als Teil einer Komponente von Flüssigkristallmischungen läßt sich für den Fachmann daraus jedoch nicht ableiten.

Es wurde nun gefunden, daß Verbindungen der Formel (I)

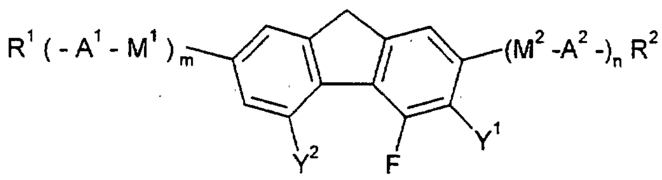

(I)

mit den Bedeutungen:

$Y^1$, $Y^2$ sind gleich oder verschieden H oder F,

$R^1$, $R^2$ sind gleich oder verschieden

Wasserstoff oder ein geradkettiger oder verzweigter Alkylrest mit 1 bis 16 C-Atomen oder ein geradkettiger oder verzweigter Alkenylrest mit 2 bis 16 C-Atomen, worin

a) eine oder mehrere nicht benachbarte und nicht terminale $CH_2$-Gruppen durch -O-, -C(=O)O-, -O-C(=O)-, -O-C(=O)-O-, -C(=O)- oder -$Si(CH_3)_2$- ersetzt sein können, und/oder
b) eine $CH_2$-Gruppe durch -C≡C-, Cyclopropan-1,2-diyl oder Cyclobutan-1,3-diyl ersetzt sein kann, und/oder
c) ein oder mehrere H-Atome durch F oder Cl ersetzt sein können, und/oder
d) die terminale $CH_3$-Gruppe durch eine der folgenden chiralen Gruppen (optisch aktiv oder racemisch) ersetzt sein kann:

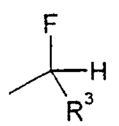
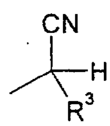
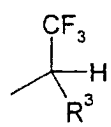
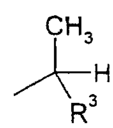

wobei $R^3$ ein geradkettiger oder verzweigter Alkylrest mit 1 bis 12 C-Atomen oder ein geradkettiger oder verzweigter Alkenylrest mit 2 bis 12 C-Atomen ist, worin eine oder mehrere nicht benachbarte und nicht terminale $CH_2$-Gruppen durch -O-ersetzt sein können und/oder ein oder mehrere H-Atome durch F und/oder Cl ersetzt sein können mit der Maßgabe, daß nur einer von $R^1$, $R^2$ gleich Wasserstoff sein kann,
$M^1$, $M^2$ sind gleich oder verschieden
-C(=O)-O-, -O-C(=O)-, -$CH_2$-O-, -O-$CH_2$-, -$CF_2$-O-, -O-$CF_2$-, -CH=CH-, -CF=CF-, -C≡C-, - -$CH_2$-$CH_2$-C(=O)-O-, -O-C(=O)-$CH_2$-$CH_2$-, -$CH_2$-$CH_2$-, -$CF_2$-$CF_2$-, -$(CH_2)_4$- oder eine Einfachbindung,
$A^1$, $A^2$ sind gleich oder verschieden
1,4-Phenylen, worin ein oder zwei H-Atome durch F, Cl oder CN ersetzt sein können, Pyridin-2,5-diyl, worin ein H-Atom durch F ersetzt sein kann, Pyrimidin-2,5-diyl, 1,4-Cyclohexylen, worin ein oder zwei H-Atome durch $CH_3$ oder F ersetzt sein können, 1-Cyclohexen-1,4-diyl, worin ein H-Atom durch $CH_3$ oder F ersetzt sein kann, oder 1,3-Dioxan-2,5-diyl,
m, n sind unabhängig voneinander 0 oder 1, in Summe jedoch nicht größer als 1,
diesen Anforderungen in besonderer Weise genügen.

Liegen an mehreren der genannten Positionen Ersetzungen vor, so sind sie voneinander unabhängig aus den genannten Ersetzungen ausgewählt, und können daher gleich oder verschieden sein. Es können damit in der Verbindung auch mehrere unterschiedliche der genannten Ersetzungen an unterschiedlichen Positionen gleichzeitig vorliegen.

Bevorzugt sind die Verbindungen der Formel (I) mit den Bedeutungen:

$Y^1$ ist gleich F,

$R^1$, $R^2$ sind gleich oder verschieden
ein geradkettiger oder verzweigter Alkylrest mit 1 bis 14 C-Atomen oder ein geradkettiger oder verzweigter Alkenylrest mit 2 bis 14 C-Atomen, worin eine oder zwei nicht benachbarte und nicht terminale $CH_2$-Gruppen durch

-O- ersetzt sein können und/oder ein oder mehrere H-Atome durch F ersetzt sein können,

$R^3$ ist ein geradkettiger oder verzweigter Alkylrest mit 1 bis 10 C-Atomen, worin eine nicht terminale $CH_2$-Gruppe durch -O- ersetzt sein kann,

$M^1$, $M^2$ sind gleich oder verschieden
$-CH_2-O-$, $-O-CH_2-$, $-CH_2-CH_2-$ oder eine Einfachbindung,

$A^1$, $A^2$ sind gleich oder verschieden
1,4-Phenylen, worin ein oder zwei H-Atome durch F ersetzt sein können, oder 1,4-Cyclohexylen,

m, n sind unabhängig voneinander 0 oder 1, in Summe jedoch nicht größer als 1.

Besonders bevorzugt sind die folgenden Verbindungen der Formeln (I a) bis (I p)

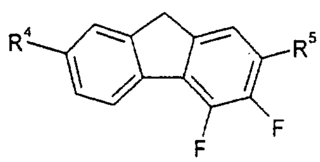

(I a)

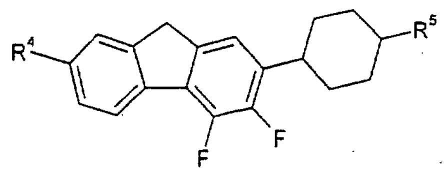

(I b)

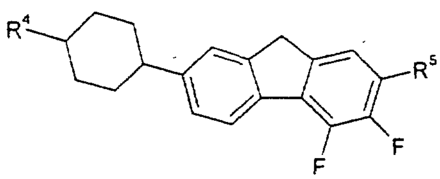

(I c)

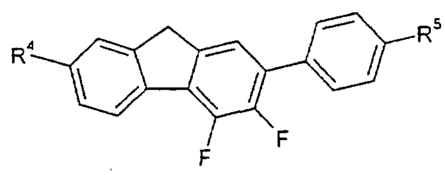

(I d)

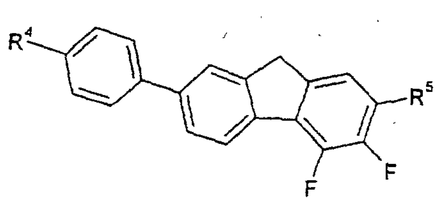

(I e)

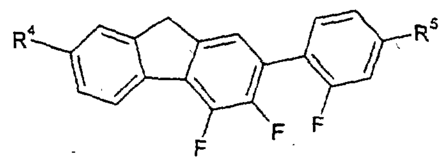

(I f)

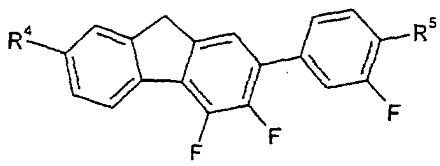

(I g)

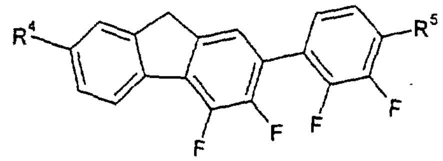

(I h)

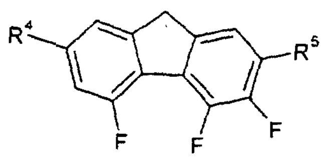

(I i)

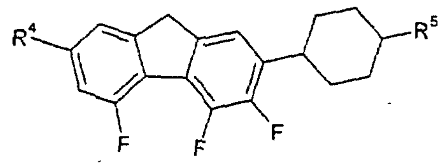

(I k)

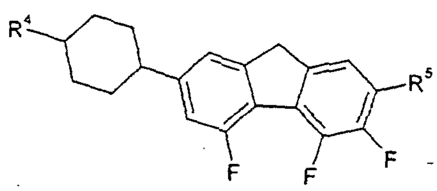

(I l)

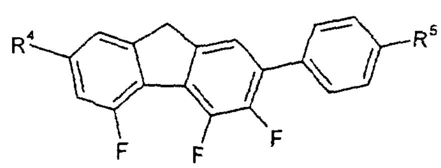

(I m)

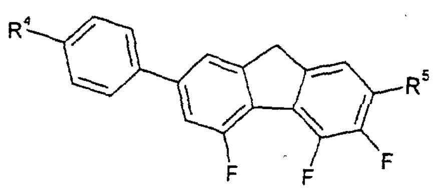

(I n)

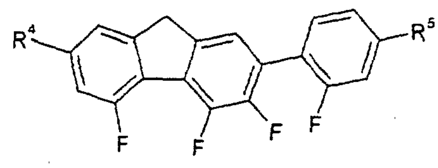

(I o)

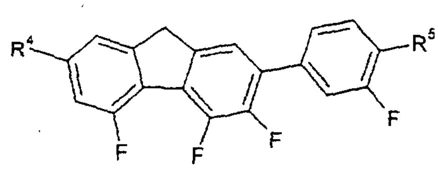

(I p)

mit den Bedeutungen:

$R^4$ und $R^5$ sind gleich oder verschieden ein geradkettiger Alkyl-, Alkenyl- oder Alkoxyrest mit 2 bis 12 C-Atomen.

Ganz besonders bevorzugt sind Verbindungen der Formeln (I a) bis (I e) und (I i) bis (I n) mit den Bedeutungen:

$R^4$, $R^5$ sind gleich oder verschieden ein geradkettiger Alkyl-, Alkenyl- oder Alkoxyrest mit 2 bis 10 C-Atomen.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich erweitert.

In diesem Zusammenhang besitzen die Verbindungen der Formel (I) einen breiten Anwendungsbereich. In Abhängigkeit von der Auswahl der Substituenten können sie anderen Verbindungsklassen zugesetzt werden, um beispielsweise die dielektrische und/oder optische Anisotropie eines solchen Dielektrikums zu beeinflussen. Auch können sie dazu dienen, dessen Schwellenspannung und/oder dessen Viskosität zu optimieren.

Besonders geeignet sind die Verbindungen der Formel (I), um schon in geringen Zumischmengen die dielektrische Anisotropie ($\Delta\varepsilon$) in Richtung auf höhere negative Werte zu beeinflussen.

Gegenstand der Erfindung ist somit neben Verbindungen der Formel (I) auch die Verwendung dieser Verbindungen als Komponenten flüssigkristalliner Mischungen.

Die Verbindungen der Formel (I) können in Flüssigkristallmischungen eingesetzt werden. Sie eignen sich im Falle von nematischen Mischungen besonders für "Active Matrix Displays" (AM-LCD) (siehe z. B. C. Prince, Seminar Lecture Notes, Volume I, p. M-3/3-M-22, SID International Symposium 1997, B. B: Bahadur, Liquid Crystal Applications and Uses, Vol. 1, p. 410, World Scientific Publishing, 1990, E. Lüder, Recent Progress of AMLCD's, Proceedings of the 15th International Display Research Conference , 1995, p. 9-12) und "in-plane-switching Displays" (IPS-LCD), im Falle von smektischen Flüssigkristallmischungen für geneigt smektische (ferroelektrische oder antiferroelektrische) Displays. Im Fall von ferroelektrischen Mischungen eignen sich die erfindungsgemäßen Verbindungen der Formel (I) insbesondere zur Verwendung in Displays, die im Inverse-Mode (siehe z. B.: J. C. Jones, M. J. Towler, J. R. Hughes, Displays 1993, 14 , Nr. 2, 86-93; M. Koden, Ferroelectrics 1996, 179, 121-129) betrieben werden.

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. In diesem Sinne geeignete Mischungskomponenten sind insbesondere in WO 00/36054, WO 97/04039, DE-A-195 31 165 sowie EP-A-0 893 424 aufgeführt, auf die hiermit ausdrücklich Bezug genommen wird.

Gegenstand der Erfindung sind auch Flüssigkristallmischungen, die mindestens eine Verbindung der Formel (I), vorzugsweise in einer Menge von 1 bis 40 Gew.-%, bezogen auf die Flüssigkristallmischung, enthalten. Vorzugsweise enthalten die Mischungen mindestens 3 weitere Komponenten bzw. (flüssigkristalline) Verbindungen. Gegenstand der Erfindung sind darüber hinaus auch elektrooptische Anzeigeelemente (Flüssigkristallanzeigen, Displays), die die erfindungsgemäßen Mischungen enthalten.

Bevorzugt sind Flüssigkristallanzeigen, die die erfindungsgemäßen nematischen oder smektischen (ferroelektrischen oder antiferroelektrischen) Mischungen in Kombination mit Aktiv-Matrix-Elementen enthalten.

Ebenfalls bevorzugt sind Flüssigkristallanzeigen, die die erfindungsgemäßen ferroelektrischen Flüssigkristallmischungen enthalten und im sogenannten Inverse- oder $\tau V_{(min)}$-Mode betrieben werden.

Die erfindungsgemäßen Anzeigeelemente (Displays) sind üblicherweise so aufgebaut, daß eine Flüssigkristallschicht beiderseitig von Schichten eingeschlossen ist, die üblicherweise, in dieser Reihenfolge, ausgehend von der LC-Schicht, mindestens eine Orientierungsschicht, Elektroden und eine Begrenzungsscheibe (z. B. aus Glas) sind. Darüber hinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z. B. E. Kaneko, "Liquid Crystal TV Displays: Principles and Applications of Liquid Crystal Displays", KTK Scientific Publishers, 1987).

Die Schaltcharakteristika einer FLC-Vorrichtung können im allgemeinen durch ein Diagramm dargestellt werden, bei dem die Treiberspannung (V) horizontal und die Breite der Ansteuerpulse ($\tau$, Zeit) vertikal aufgetragen sind (siehe z. B. J. C. Jones, M. J. Towler, J. R. Hughes, Displays 1993, 14 , Nr. 2, 86-93, Abb. 4, 8, 10 und 11). Eine Schaltkurve wird experimentell bestimmt und teilt die V, $\tau$-Fläche in einen schaltenden und einen nicht schaltenden Bereich. Üblicherweise verkürzt sich bei Erhöhung der Spannung die Pulsbreite. Dieses Verhalten kennzeichnet den sogenannten Normal-Mode (siehe z. B. Jones, Abb. 4). Bei geeigneten Materialien weist die $V\tau$-Kurve jedoch ein Minimum auf (bei der Spannung $V_{(min)}$), wie z. B. bei Jones in den Abbildungen 8, 10 und 11 zu sehen. Dieses Minimum kommt durch Überlagerung von dielektrischer und ferroelektrischer Verdrillung zustande. FLC-Vorrichtungen werden im Inverse-Mode betrieben, wenn die Summe der Zeilen- und Spalten-Treiberspannung im Arbeitstemperaturbereich

höher als das Minimum auf der Vτ-Kurve sind, d. h. $V_{(Zeile)} + V_{(Spalte)} > V_{(min)}$.

Beispielhaft sind in den nachfolgenden Schemata 1 bis 7 mögliche Synthesewege zu Verbindungen der Formel (I) angegeben, wobei auch andere Verfahren denkbar und möglich sind.

Folgende Abkürzungen werden verwendet:

| | |
|---|---|
| *n*-BuLi | *n*-Buthyllithium |
| DCC | Dicyclohexylcarbodiimid |
| DEAD | Diethylazodicarboxylat (Azodicarbonsäurediethylester) |
| DMAP | 4-(Dimethylamino)pyridin |
| DME | Dimethoxyethan |
| DMF | N,N-Dimethylformamid |
| KO*t*Bu | Kalium-*tert*-butylat |
| LiTMP | Lithium-2,2,6,6-tetramethylpiperidid |
| MTBE | *tert*-Butylmethylether |
| 4-TsOH | 4-Toluolsulfonsäure |

Schema 1

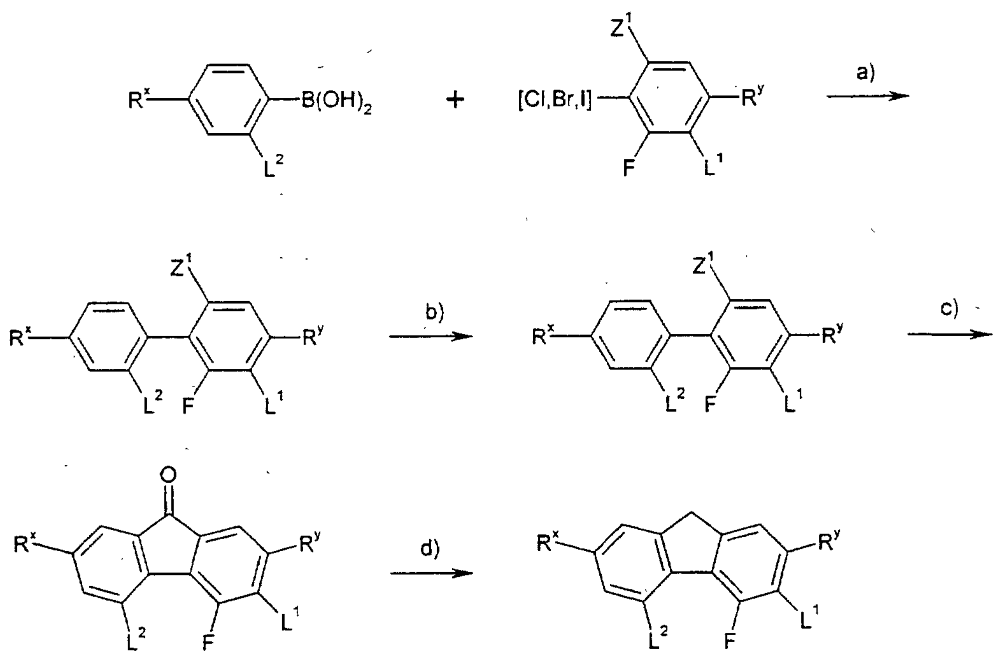


$Z^1$ = CHO, CN, COOH

a) $Pd(PPh_3)_4/CsF/DME$ ($Z^1$ = CHO) bzw. $Pd(PPh_3)_4/Na_2CO_3$/Toluol/EtOH/$H_2O$ ($Z^1$ = CN) bzw. $Pd(PPh_3)_4/Na_2CO_3/CH_3CN/H_2O$ ($Z^1$ = COOH)

b) $KMnO_4$/Aceton/$H_2O$ ($Z^1$ = CHO) bzw. $H_2SO_4/H_2O$ ($Z^1$ = CN)

c) 1. $SOCl_2$ 2. $AlCl_3/CH_2Cl_2$ d) $Et_3SiH/F_3CCOOH$

Dabei können die Synthesen der mesogenen Boronsäuren der einschlägigen Literatur (z.B. Gray, Mol.Cryst. Liq.Cryst. 204, 43 (1991)) entnommen werden. Die Synthesen der Halogen-Partner in der Suzuki-Kupplungs-Reaktion gemäß Schema 1 können z.B. in Analogie zu Li, Org.Prep.Proc.Int. 28, 245 (1996) (dort Synthese von 2-Chlor-4,5-difluorbenzoesäure) erfolgen; für die Halogen-Komponenten der Suzuki-Reaktion gemäß Schema 2 wird auf Gray et al., Mol.Cryst.Liq.Cryst. 204, 43 (1991) verwiesen; in dieser Literaturstelle wird u.a. die Synthese von 4-Alkyl- bzw. 4-Alkyloxy-2-fluor-brombenzolen beschrieben.

Schema 2

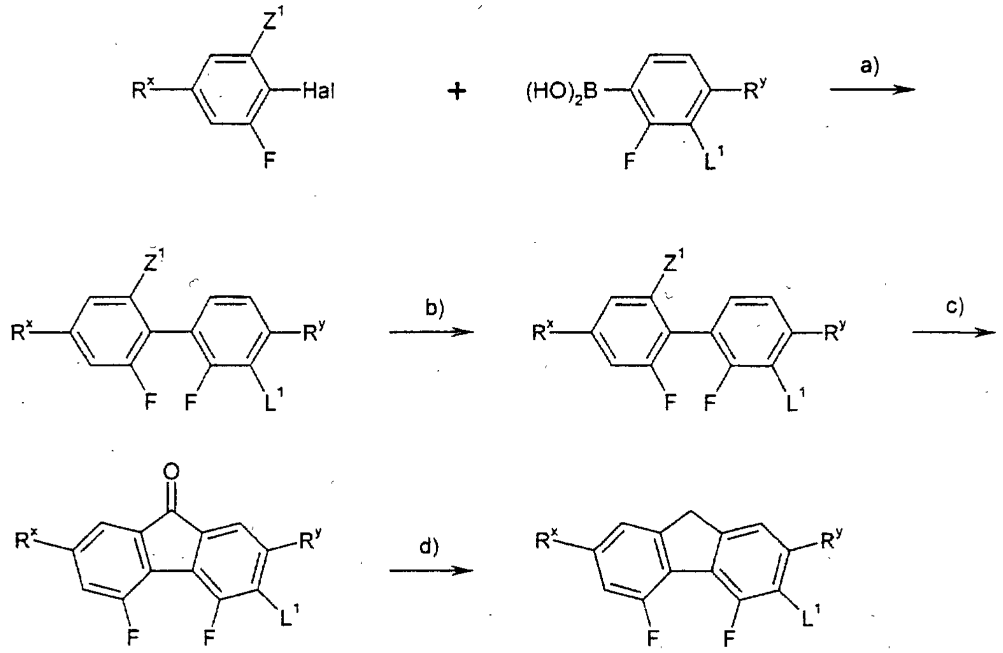


$Z^1$ = $CONR_2$, CN, COOH, CHO

a) $Pd(PPh_3)_4/CsF/DME$ ($Z^1$ = CHO) bzw. $Pd(PPh_3)_4/Na_2CO_3$/Toluol/EtOH/$H_2O$ ($Z^1$ = CN) bzw. $Pd(PPh_3)_4/Na_2CO_3/CH_3CN/H_2O$ ($Z^1$ = COOH)

b) $KMnO_4$/Aceton/$H_2O$ ($Z^1$ = CHO) bzw. $H_2SO_4/H_2O$ ($Z^1$ = CN)

c) 1. $SOCl_2$ 2. $AlCl_3/CH_2Cl_2$ d) $Et_3SiH/F_3CCOOH$

In den Schemata 1 bis 8 bedeutet die Gruppe $R^x$ die Gruppierung $R^1(-A^1-M^1)_m$ oder eine geeignete, gegebenenfalls geschützte Vorstufe hiervon (beispielsweise Br oder $OSO_2CF_3$ im Fall Hal = I; Benzyloxy; Tetrahydopyranyloxy; *tert*-Butyldiphenylsilanyloxy), die in späteren Schritten nach an sich bekannten, dem Fachmann geläufigen Methoden in diese Gruppe überführt werden kann. Entsprechend bedeutet die Gruppe $R^y$ die Gruppierung $(M^2-A^2-)R^2$ oder eine geeignete, gegebenenfalls geschützte Vorstufe hiervon. Die Gruppe Hal bedeutet Cl, Br, I oder $OSO_2CF_3$.

$R^x$ und $R^y$ können in diesem Sinne auch Wasserstoff bedeuten, da z.B. gemäß Schema 5 und 6 *ortho*-Metallierung und damit Folgereaktionen an diesen Positionen möglich sind.

Beispielsweise können nach Schema 1 und 2 erhaltene 7-methoxy- und 7-bromsubstituierte Fluorene ($R^x$ = Br oder $H_3CO$) gemäß Schema 3 und 4 zu den erfindungsgemäßen Verbindungen der Formel (I) umgesetzt werden.

Schema 3

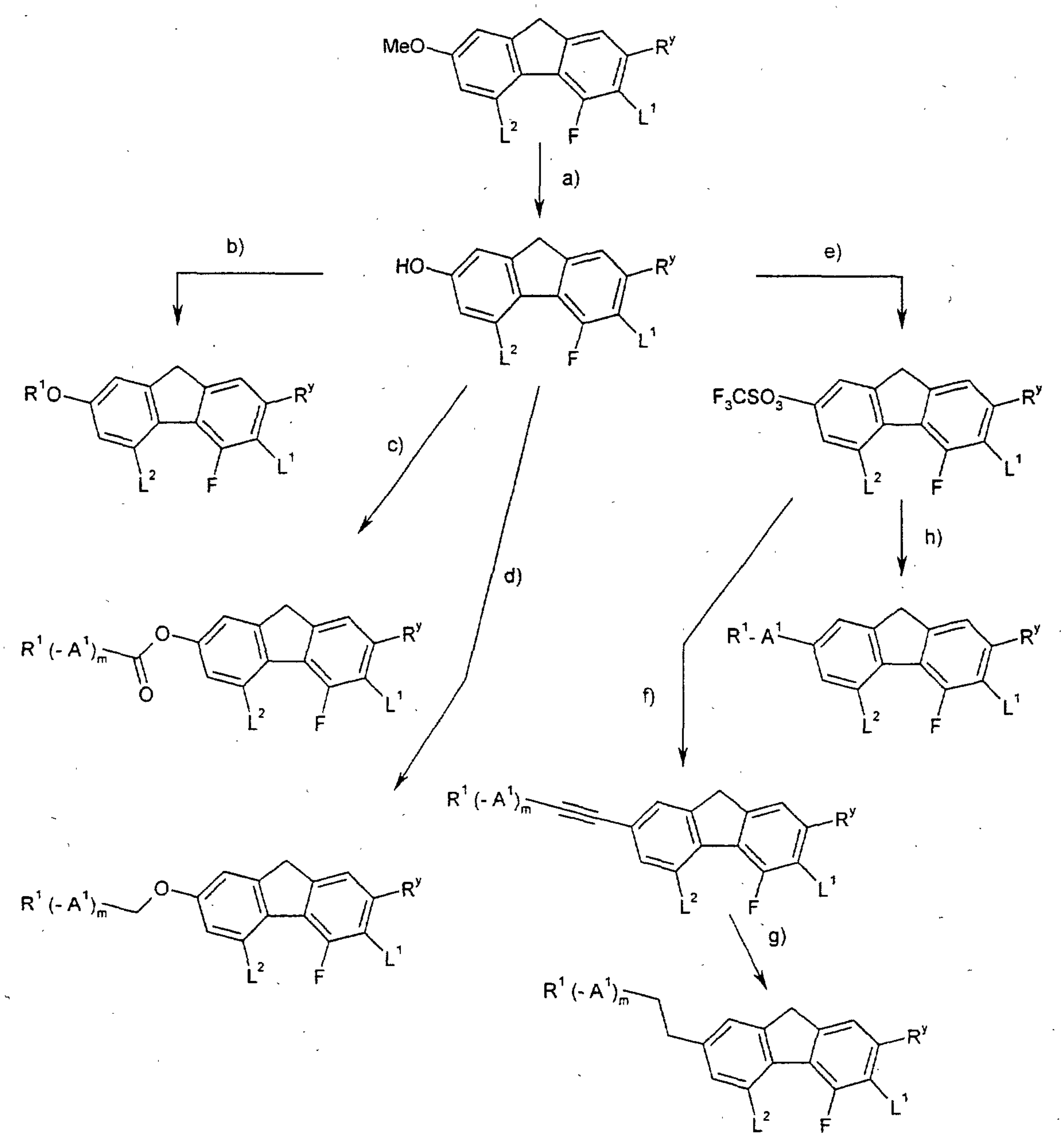


a) $HBr/CH_3COOH$ b) $R^1Br/K_2CO_3$/MEK c) $R^1(-A^1)_m$-COOH/DCC/DMAP/ $CH_2Cl_2$ d) $R^1(-A^1)_m$-$CH_2OH$/DEAD/$PPh_3$/THF e) $(F_3CSO_2)_2O$ / Pyridin

f) $R^1(-A^1)_m$-C≡CH/$Pd(PPh_3)_2Cl_2$/ CuI/$NEt_3$ g) $H_2$/Pd-C/THF

h) $R^1$-$A^1$-$B(OH)_2$/$Pd(PPh_3)_4$/$Na_2CO_3$/Toluol/EtOH/$H_2O$

Schema 4

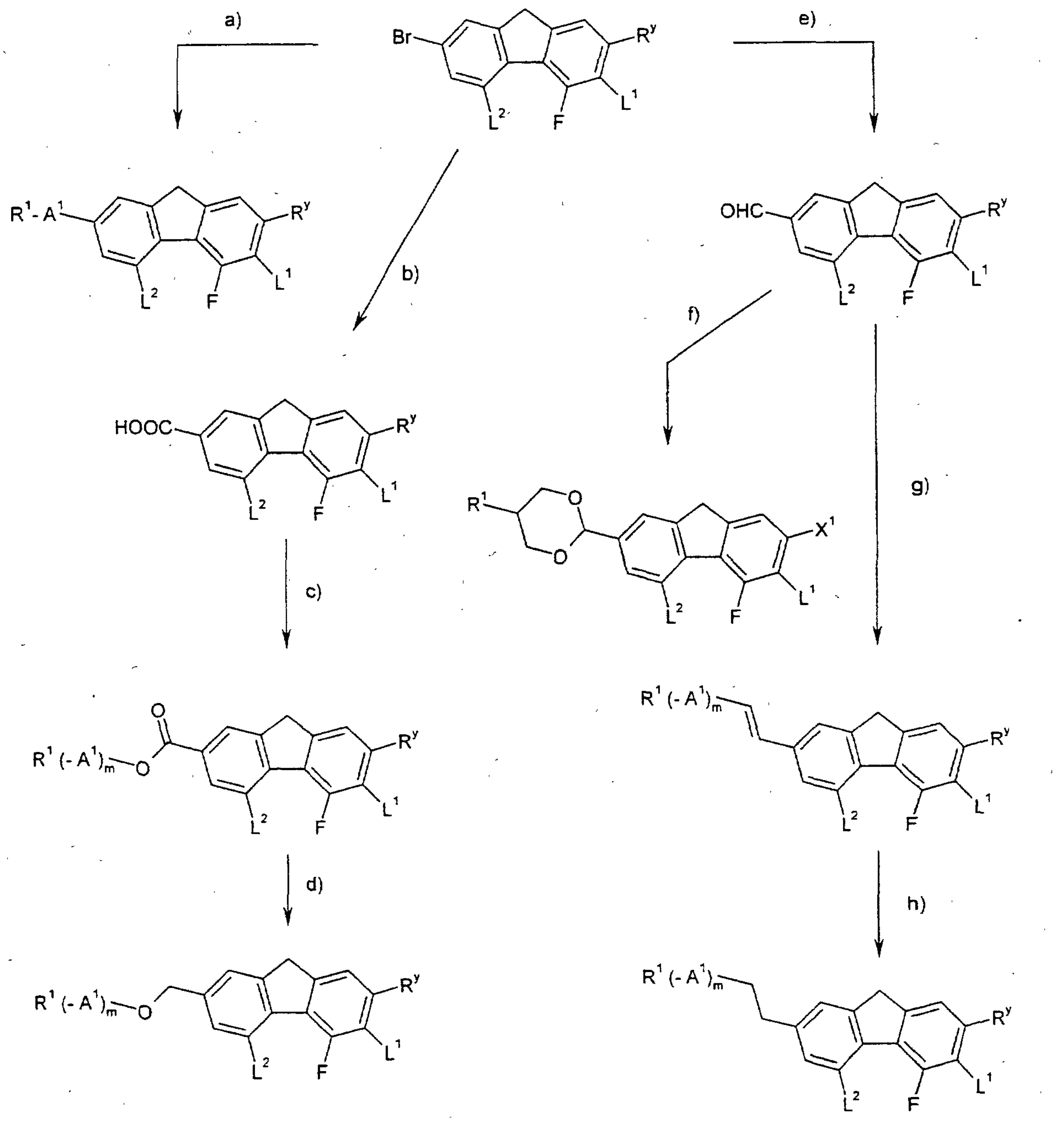


a) $R^1$-$A^1$-$B(OH)_2$/$Pd(PPh_3)_4$/$Na_2CO_3$/Toluol/EtOH/$H_2O$

b) 1. Mg/THF 2. $CO_2$ 3. $H_3O^+$ c) $R^1(-A^1)_m$-OH/DCC/DMAP/$CH_2Cl_2$

d) $NaBH_4$/$BF_3$/THF/Diglyme e) Mg/THF 2. DMF 3. $H_3O^+$

f) $R^1$-$CH(CH_2OH)_2$/4-TsOH/Toluol

g) $R^1(-A^1)_m$-$CH_2$-$P(Ph_3)Br$/KO*t*Bu/THF h) $H_2$/Pd-C/THF

In Position 2 durch geeignete $R^x$ substituierte 3,4-Difluorfluorene können durch Metallierung der Position 7 (analog: Synlett 1990, 747, ibid. 1991, 119, Tetrahedron Lett. 1996, 37, 6551) und nachfolgende Umsetzung mit Elektrophilen (z. B. Trimethylborat, DMF, $CO_2$, *n*-Alkylaldehyden, 4-*n*-Alkylcyclohexanonen, Iod, Brom) zu Vorstufen umgesetzt werden, aus denen sich die erfindungsgemäßen Verbindungen der Formel (I) herstellen lassen (Schemata 5 bis 8).

Schema 5

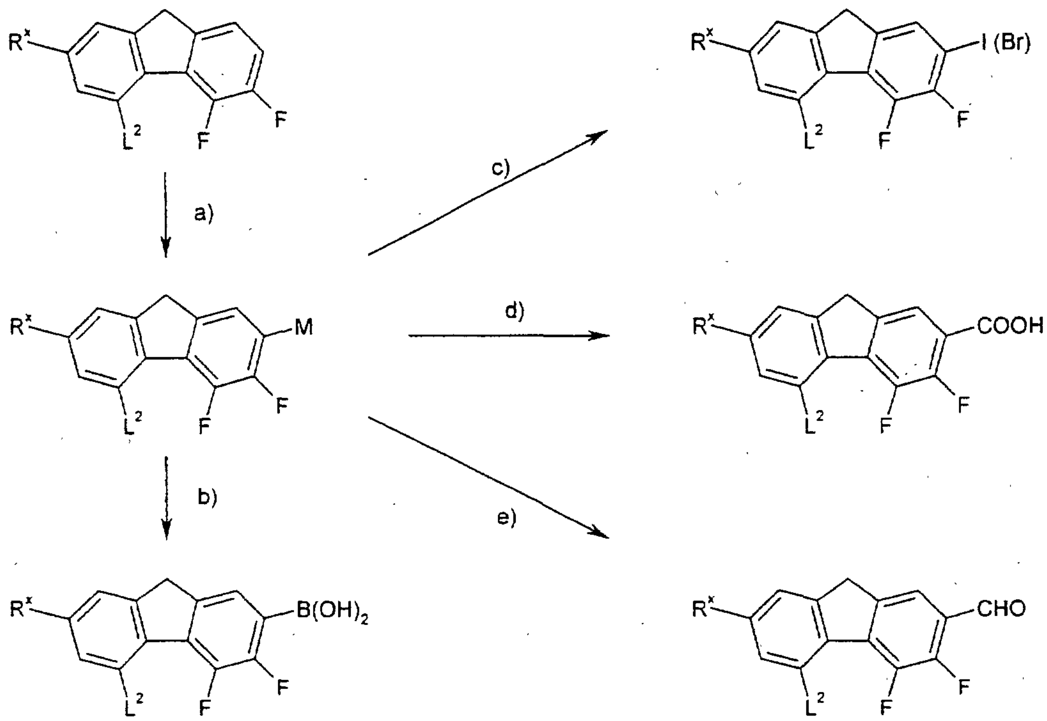


M = Li, K a) *n*-BuLi/THF/Hexan oder *n*-BuLi/KO*t*Bu/THF/Hexan oder *sec*-BuLi/THF/Cyclohexan oder LiTMP/THF/Hexan b) 1. $B(OMe)_3$ 2. $H_3O^+$
c) $I_2$ (bzw. $Br_2$) d) 1. $CO_2$ 2. $H_3O^+$ e) 1. DMF 2. $H_3O^+$

Schema 6

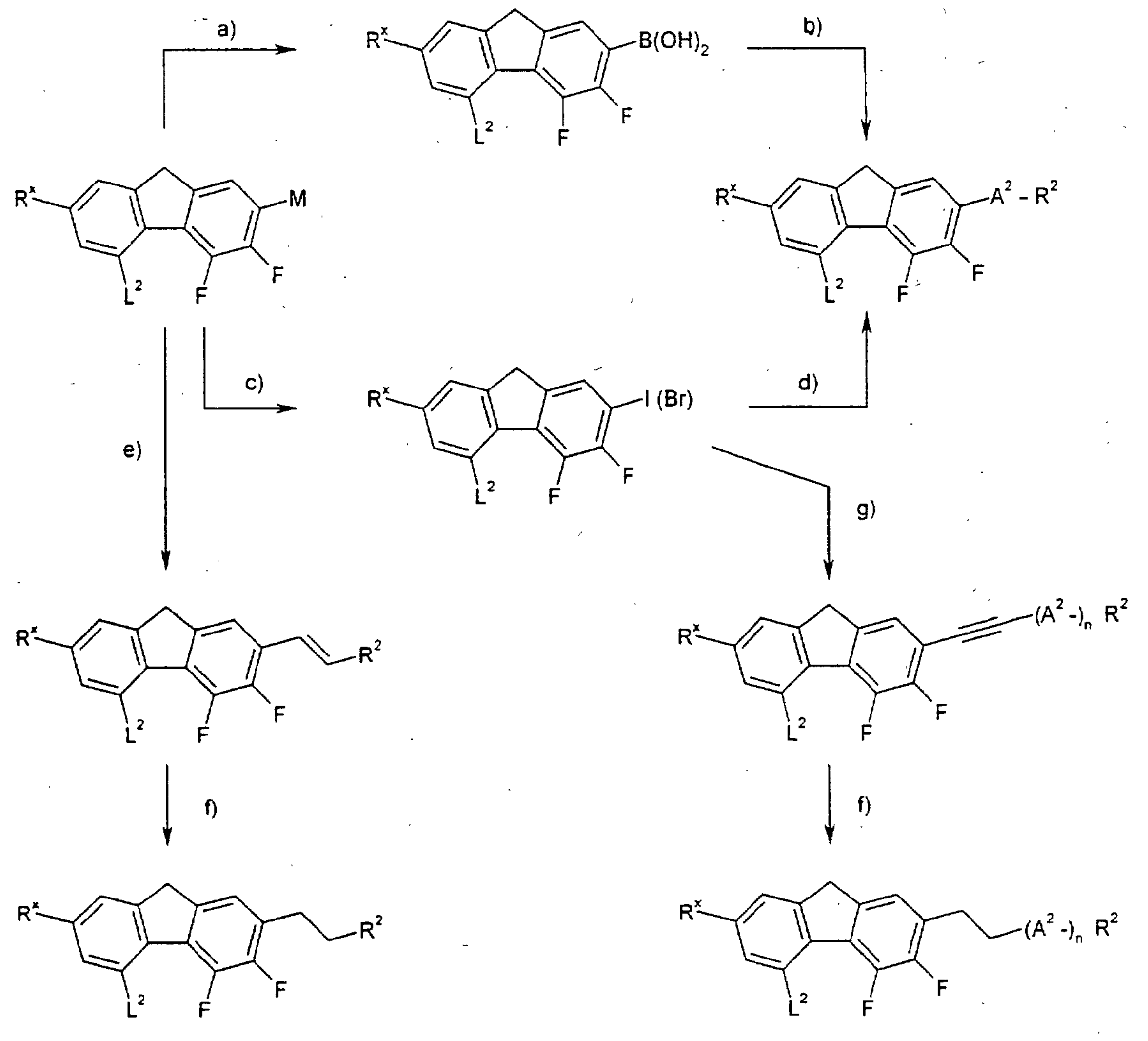


a) 1. $B(OMe)_3$ 2. $H_3O^+$ b) $R^2$-$A^2$-Hal/$Pd(PPh_3)_4$/$Na_2CO_3$/Toluol/EtOH/$H_2O$

c) $I_2$ (oder $Br_2$) d) $R^2$-$A^2$-$B(OH)_2$/$Pd(PPh_3)_4$/$Na_2CO_3$/Toluol/EtOH/$H_2O$

e) 1. $R^2$-$CH_2CHO$ 2. $H_3O^+$ 3. 4-TsOH/Toluol f) $H_2$/Pd-C/THF

g) $R^2$-$(A^2)_n$-C≡CH/$Pd(PPh_3)_2Cl_2$/CuI/$NEt_3$

Schema 7

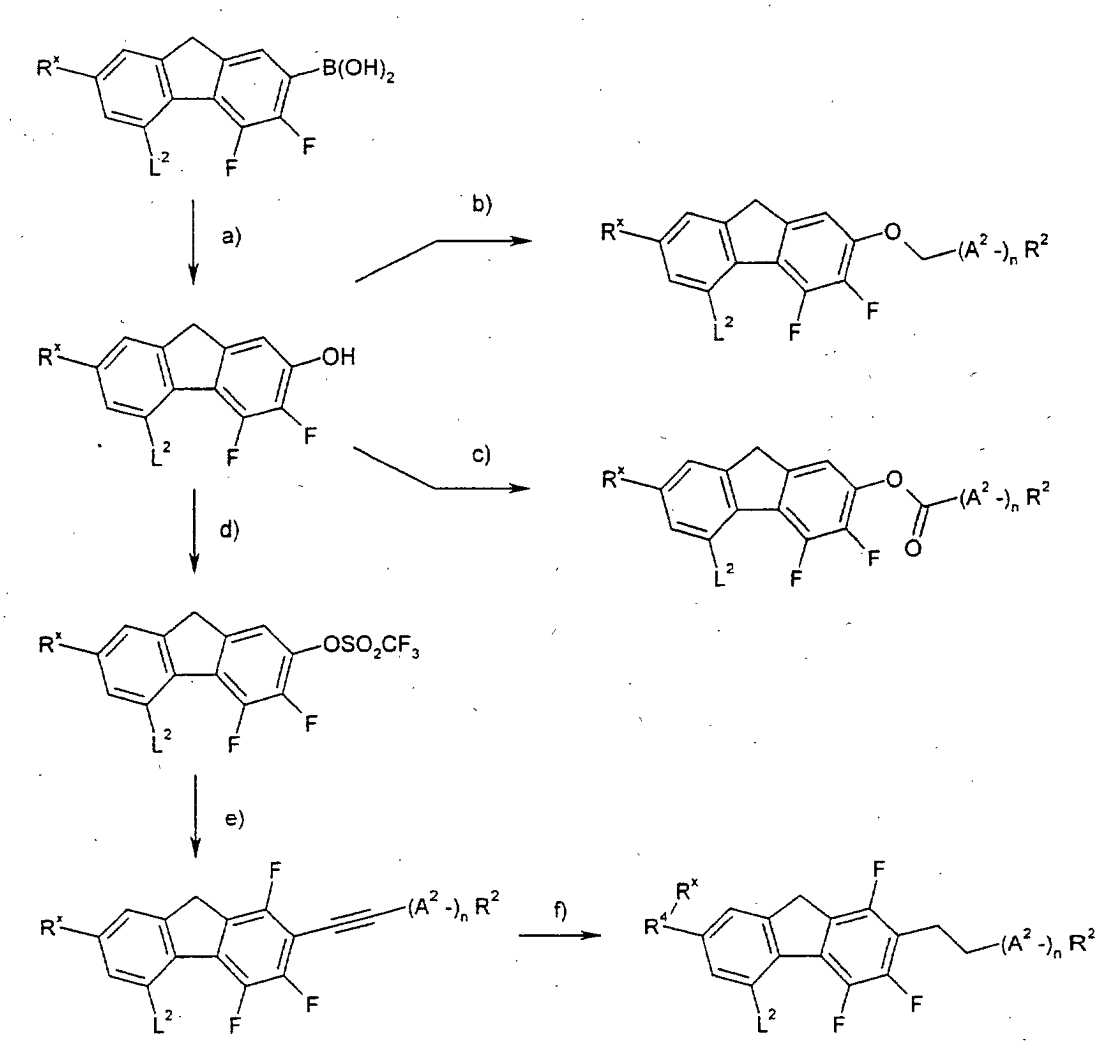


a) $H_2O_2$/MTBE b) $R^2(-A^2)_n-CH_2OH$/DEAD/$PPh_3$/THF oder
$R^2(-A^2)_n-CH_2Br$/$K_2CO_3$/MEK c) $R^2(-A^2)_n-COOH$/DCC/DMAP/ $CH_2Cl_2$
d) $(F_3CSO_2)_2O$/Pyridin e) $R^2(-A^2)_n-C{\equiv}CH$/$Pd(PPh_3)_2Cl_2$/CuI/$NEt_3$
f) $H_2$/Pd-C/THF

Schema 8

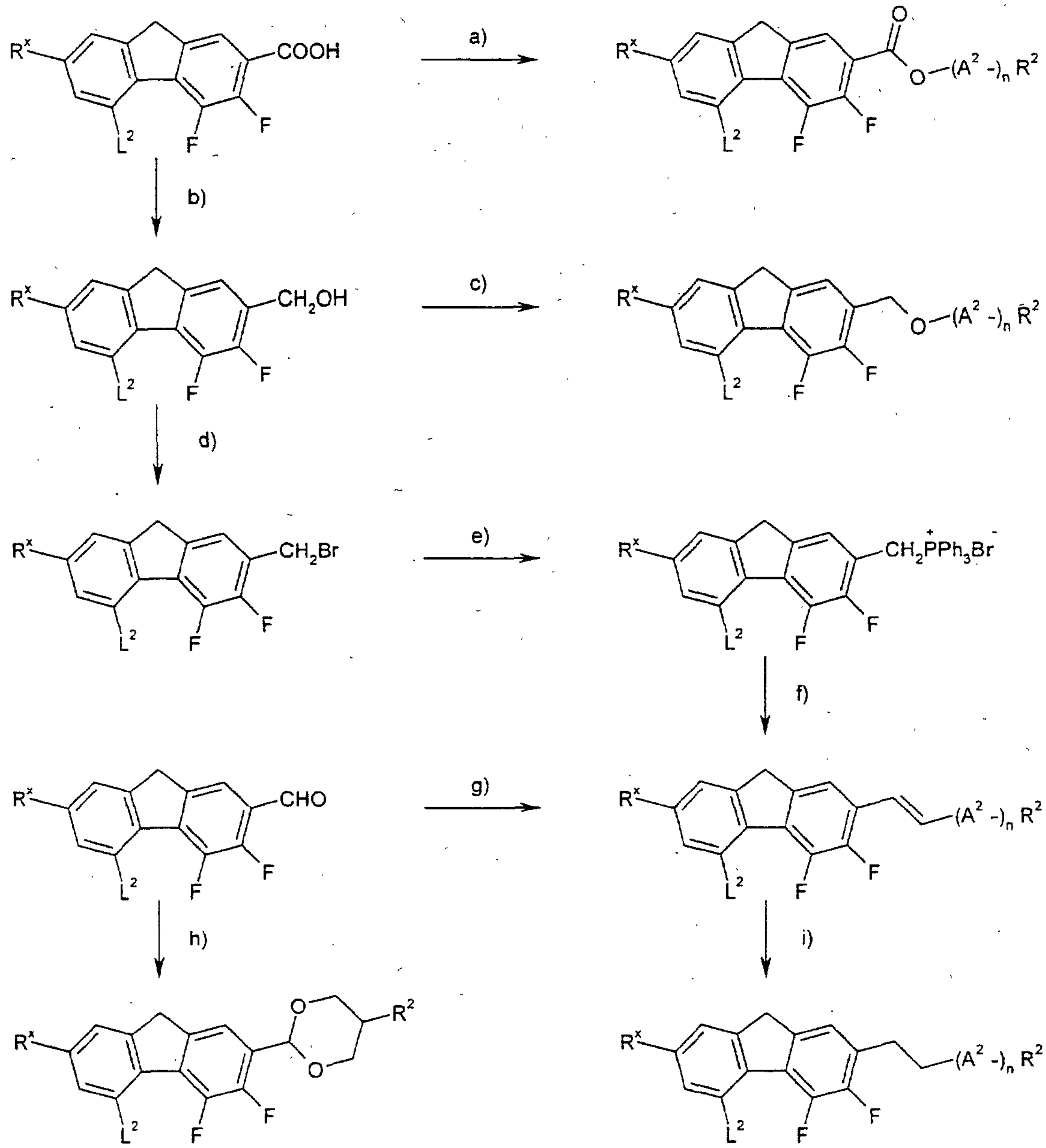


a) $R^2(-A^2)_n$-OH/DCC/DMAP/$CH_2Cl_2$ b) $LiAlH_4$/THF 2. $H_3O^+$

c) $R^2(-A^2)_n$-OH/DEAD/$PPh_3$/THF d) $PBr_3/CHCl_3$ e) $PPh_3$/Xylol

f) $R^2(-A^2)_n$-CHO/KO*t*Bu/THF g) $R^2(-A^2)_n$-$CH_2P(Ph_3)Br$/KO*t*Bu/THF

h) $R^2$-$CH(CH_2OH)_2$/4-TsOH/Toluol i) $H_2$/Pd-C/THF

Die Herstellung der für die Synthesen nach Schemata 1 bis 8 benötigten, beispielsweise alkyl-, alkenyl- oder alkoxy-substituierten, ggf. zusätzlich fluorierten Benzoesäuren, Cyclohexancarbonsäuren und Phenylacetylene (Schemata 3,6 und 7), Phenylboronsäuren, Pyridylboronsäuren, Brombenzole, Brompyrimidine , Cyclohexanone und 2-Alkylpropan-1,3-diole (Schema 8) und deren Umsetzungen sind dem Fachmann bekannt und beispielsweise in WO 96/00710, WO 96/30344, Liq. Cryst. 1995, 18, 1, Mol. Cryst. Liq. Cryst. 1991, 204, 43, Liq. Cryst. 1997, 23, 389, Synthesis 1996, 589, WO 92/11241, EP-A-0 665 825 und J. Mater. Chem. 1999, 9, 1669 beschrieben. Entsprechend substituierte Benzylalkohole und (Hydroxymethyl)-cyclohexane $R^2$-$A^2$-$CH_2OH$ lassen sich beispielsweise aus den ent-

sprechenden Benzoesäuren bzw. Cyclohexancarbonsäuren $R^2$-$A^2$-COOH durch Reduktion mit Lithiumaluminiumhydrid (allg. Arbeitsvorschrift: Organikum, VEB Deutscher Verlag der Wissenschaften, 15. Aufl., Berlin 1984, Kap. 7.3.4) erhalten. Deren Bromierung mit Phosphortribromid (analog J. Org. Chem. 1984, 49, 2534-2540) liefert die in Schema 5 benötigten Benzylbromide bzw. (Brommethyl)-cyclohexane $R^2$-$A^2$-$CH_2$Br. Anschließende Umsetzung mit Triphenylphosphin in Xylol liefert die in Schema 4 erwähnten Triphenylphosphoniumbromide $R^2$-$A^2$-$CH_2$-P($Ph_3$)Br. Entsprechend substituierte Benzaldehyde und Cyclohexancarboxaldehyde $R^2$-$A^2$-CHO sind beispielsweise durch Reduktion des jeweiligen Carbonsäureesters $R^2$-$A^2$-COOR (Bull. Korean. Chem. Soc. 1999, 20, 1373) oder Oxidation der zuvor genannten Benzylalkohole und (Hydroxymethyl)-cyclohexane $R^2$-$A^2$-$CH_2$OH (Tetrahedron Lett. 1968, 30, 3363) erhältlich.

Erfindungsgemäße Verbindungen der Formel (I) mit 1-Cyclohexen-1,4-diyl oder 2-Fluor-1-cyclohexen-1,4-diyl- oder 4-Fluor-3-cyclohexen-1-yl-Einheit werden wie in Liq. Cryst. 1997, 23, 69, DE-A-44 27 266, DE-A-196 07 996, DE-A-195 28 665 und EP-A-0 736 513 beschrieben hergestellt. Was die Synthese spezieller Reste $R^1$ und $R^2$ angeht, sei zusätzlich beispielsweise verwiesen auf EP-A-0 355 008 (für Verbindungen mit siliciumhaltigen Seitenketten), US 4,798,680 (für optisch aktive Verbindungen mit 2-Fluoralkyloxy-Einheit), EP-A-0 552 658 (für Verbindungen mit Cyclohexylpropionsäureresten) und EP-A-0 318 423 (für Verbindungen mit Cyclopropylgruppen in der Seitenkette).

Die Erfindung wird durch die nachstehenden Beispiele näher erläutert.

**Beispiel 1:**

**7-Pentyl-2-propyl-3,4-difluor-fluoren**

Zu 6,0g (20 mmol) 5,6-Difluor-4'-pentyl-biphenyl-2-carbonsäure (erhalten durch Suzuki-Reaktion aus 4-Pentylphenylboronsäure [121219-12-3] und 2-Chlor-4,5-difluorbenzoesäure (Org.Prep.Proc.Int. 28, 245 (1996)) werden 20 ml Thionylchlorid gegeben. Die Mischung wird bis zum Ende der Gasentwicklung zum Sieden erhitzt. Nach dem Abkühlen wird überschüssiges Thionylchlorid im Vakuum abdestilliert. Eine Lösung des erhaltenen Säurechlorids in 50 ml Dichlormethan wird unter Rühren und Kühlen langsam innerhalb 1 h bei 0 bis 5 °C zu einer Suspension von 2,9 g (22 mmol) Aluminiumchlorid in 200 ml Dichlormethan getropft. Anschließend wird noch 1 h bei Raumtemperatur nachgerührt. Man gibt die Reaktionsmischung auf Eis und bringt ausgeschiedenes Aluminiumhydroxid mit konzentrierter Salzsäure in Lösung. Die organische Phase wird mit Wasser und verdünnter Natronlauge gewaschen, mit Natriumsulfat getrocknet, und das Lösemittel wird im Vakuum entfernt. Das Rohprodukt reinigt man säulenchromatographisch (Kieselgel, Dichlormethan) und durch Umkristallisation. Man erhält 3,4-Difluor-7-pentyl-fluoren-9-on in Form leicht gelblicher Kristalle.

4 g (14 mmol) 3,4-Difluor-7-pentyl-fluoren-9-on werden in 30 ml Trifluoressigsäure gelöst und 6,2 g (52 mmol) Triethylsilan werden langsam bei Raumtemperatur zugetropft. Man rührt anschließend 6 h unter Rückfluß. Nach dem Abkühlen wird das Reaktionsgemisch auf Eis/Wasser gegeben. Man trennt die Phasen, extrahiert die wässrige Phase mit *tert*-Butylmethylether, wäscht die vereinigten organischen Phasen mit Wasser und verdünnter Natriumhydrogencarbonatlösung und trocknet mit Natriumsulfat. Das Lösemittel wird im Vakuum entfernt, und das Rohprodukt wird säulenchromatographisch an Kieselgel (*n*-Heptan) gereinigt. Nach Umkristallisation aus *n*-Heptan erhält man 3,4-Difluor-7-pentylfluoren in Form farbloser Kristalle.

Eine Lösung von 15 mmol *n*-Butyllithium (1,6 M Lösung in Hexan) in 20 ml trockenem Tetrahydrofuran wird auf ≤ -70°C gekühlt und unter Schutzgasatmosphäre tropfenweise mit einer Lösung von 15 mmol Kalium-*tert*-butylat in 20 ml des gleichen Lösemittels versetzt. Nach 15-30 min Rühren tropft man eine Lösung von 13 mmol 3,4-Difluor-7-pentyl-fluoren in 30 ml Tetrahydrofuran so zu, daß die Innentemperatur stets unterhalb -70°C liegt. Anschließend rührt man noch 2 h bei dieser Temperatur nach. Nun tropft man 15 mmol Propionaldehyd bei ≤ -70°C zu und läßt langsam auftauen. Die Reaktionsmischung wird bei -10°C mit 50 ml Wasser versetzt und mit konzentrierter Salzsäure pH 3 eingestellt. Man extrahiert mit *tert*-Butylmethylether, wäscht die vereinigten organischen Phasen mit gesättigter Natriumchloridlösung, trocknet mit Natriumsulfat und entfernt die Lösemittel im Vakuum. Nach Chromatografie an Kieselgel mit Dichlormethan als Eluent erhält man 3,4-Difluor-7-pentyl-2-(1-hydroxypropyl)-fluoren.

Eine Lösung von 2,1g 3,4-Difluor-7-pentyl-2-(1-hydroxypropyl)-fluoren in 30 ml Toluol wird nach Zusatz von ca. 0,1 g 4-Toluolsulfonsäure und einigen Tropfen konzentrierter Schwefelsäure 3 h am Wasserabscheider erhitzt. Nach dem Abkühlen extrahiert man mit 5%iger Natriumhydrogencarbonatlösung und gesättigter Natriumchloridlösung und trocknet mit Natriumsulfat. Anschließend werden 0,3 g Palladium-Katalysator (10 prozentig auf Aktivkohle) zugegeben, und es wird bei Raumtemperatur hydriert. Die Reaktionsmischung wird filtriert, und das Lösemittel wird im Vakuum entfernt. Die Reinigung des Rohproduktes erfolgt durch Chromatografie an Kieselgel und Umkristallisation aus Heptan. Man erhält 7-Pentyl-2-propyl-3,4-difluor-fluoren.

Analog Beispiel 1 können durch Verwendung anderer Boronsäuren im ersten Schritt der Synthesesequenz und / oder anderer Aldehyd-Komponenten in der Reaktionsstufe der ortho-Metallierung folgende Verbindungen erhalten werden:

2-Propyl-7-propyl-3,4-difluor-fluoren
2-Pentyl-7-pentyl-2-propyl-3,4-difluor-fluoren
2-Propyl-7-propyloxy-3,4-difluor-fluoren
2-Pentyl-7-pentyloxy-3,4-difluor-fluoren
7-(4-Propylcyclohexyl)-2-propyl-3,4-difluor-fluoren
7-(4-Propylphenyl)-2-propyl-3,4-difluor-fluoren
7-Pentyl-2-propyl-3,4,5-trifluor-fluoren
2-Propyl-7-propyl-3,4,5-trifluor-fluoren
2-Pentyl-7-pentyl-2-propyl-3,4,5-trifluor-fluoren
7-(4-Propylcyclohexyl)-2-propyl-3,4,5-trifluor-fluoren
7-(4-Propylphenyl)-2-propyl-3,4,5-trifluor-fluoren

Analog Beispiel 1, jedoch unter Verwendung von Trimethylborat anstelle einer Aldehyd-Komponente in der Reaktionsstufe der ortho-Metallierung und nachfolgender Hydrolyse zur entsprechenden Boronsäure, gefolgt von Suzuki-Reaktionen mit Halogen-Komponenten, können erhalten werden:

2-(4-Pentylphenyl)-7-pentyl-3,4-difluor-fluoren
2-(5-Nonyl-pyrimidin-2-yl)-7-pentyl-3,4-difluor-fluoren
2-(4-Propylcyclohexyl)phenyl-7-propyl-3,4-difluor-fluoren
2-(4-Pentylphenyl)-7-pentyl-3,4,5-trifluor-fluoren
2-(5-Nonyl-pyrimidin-2-yl)-7-pentyl-3,4,5-trifluor-fluoren
2-(4-Propylcyclohexyl)phenyl-7-propyl-3,4,5-trifluor-fluoren

Analog Beispiel 1, jedoch unter Verwendung von Trimethylborat anstelle einer Aldehyd-Komponente in der Reaktionsstufe der ortho-Metallierung und nachfolgender Hydrolyse zur entsprechenden Boronsäure, gefolgt von Oxidation zum entsprechenden 2-Hydroxyfluoren und anschließender Veretherung bzw. Veresterung, können erhalten werden:

2-Propyloxy-7-propyl-3,4-difluor-fluoren
2-Pentyloxy-7-pentyl-2-propyl-3,4-difluor-fluoren
2-Propyloxy-7-propyloxy-3,4-difluor-fluoren
2-Pentyloxy-7-pentyloxy-3,4-difluor-fluoren
7-(4-PropylcycloheXyl)-2-propyloxy-3,4-difluor-fluoren
7-(4-Propylphenyl)-2-propyloxy-3,4-difluor-fluoren
7-Pentyl-2-propyloxy-3,4,5-trifluor-fluoren
2-Propyloxy-7-propyl-3,4,5-trifluor-fluoren
2-Pentyloxy-7-pentyl-2-propyl-3,4,5-trifluor-fluoren
7-(4-Propylcyclohexyl)-2-propyloxy-3,4,5-trifluor-fluoren
7-(4-Propylphenyl)-2-propyloxy-3,4,5-trifluor-fluoren
7-propyl-3,4-difluor-fluoren-2-yl-octancarbonsäureester
7-Pentyl-3,4-difluor-fluoren-2-yl-(4-pentylcyclohexyl)carbonsäureester

Anwendungsbeispiel 1:

Eine smektisch-C-Mischung bestehend aus

| | |
|---|---|
| 5 -Octyl-2-(4-octyloxyphenyl)pyrimidin | 19,42% |
| 5-Octyloxy-2-(4-octyloxyphenyl)pyrimidin | 6,50% |
| 5-Decyl-2-(4-octyloxyphenyl)pyrimidin | 15,44% |
| 5-Octyloxy-2-(4-hexyloxyphenyl)pyrimidin | 13,11% |
| 5-Octyloxy-2-(4-butyloxyphenyl)pyrimidin | 12,82% |
| 5-Octyloxy-2-(4-decyloxyphenyl)pyrimidin | 12,61 % |
| 5-Octyl-2-(4-hexyloxyphenyl)pyrimidin | 20,10% |

weist einen Wert $\Delta\varepsilon$ der dielektrischen Anisotropie von +0.13 auf; durch Zugabe von 15% der Verbindung 2-Hexyloxy-7-hexyloxy-3,4-difluor-fluoren wird ein Wert $\Delta\varepsilon$ = -1.5 erreicht, so daß die resultierende Mischung für den Betrieb eines Displays in der sogenannten "inverse mode" (z. B. P. W. H.Surguy, Ferroelectrics 1991, 122, 63; oder P. W. Ross, Proc.

SID Conf. 1992, 217; oder J. C. Jones et al., Displays 1993, Vol.14, No.2, pp. 86-93; oder M. Koden, Ferroelectrics 1996, 179, 121) geeignet ist.

Anwendungsbeispiel 2:

Eine chiral-smektisch-C-Mischung bestehend aus

| | |
|---|---|
| 2-(4-Heptyloxyphenyl)-5-nonylpyrimidin | 19,6% |
| 5-Nonyl-2-(4-octyloxyphenyl)pyrimidin | 19,6% |
| 5-Nonyl-2-(4-nonyloxyphenyl)pyrimidin | 19,6% |
| 2-(2,3-Difluor-4-heptyloxyphenyl)-5-nonylpyrimidin | 6,5% |
| 2-(2,3-Difluor-4-octyloxyphenyl)-5-nonylpyrimidin | 6,5% |
| 2-(2,3-Difluor-4-nonyloxyphenyl)-5-nonylpyrimidin | 6,5% |
| 5-Hexyloxy-2-(4-hexyloxyphenyl)pyrimidin | 19,6% |
| (*S*)-4-[4'-(2-Fluoroctyloxy)biphenyl-4-yl]-1-heptylcyclohexancarbonitril | 2,0% |

wird mit 10% der Verbindung aus Beispiel 1 versetzt.

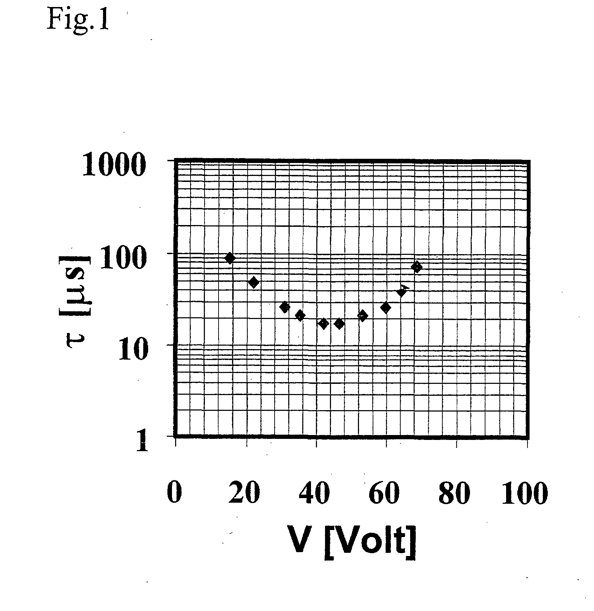

Fig. 1 zeigt die $\tau$Vmin-Kurve ($\tau$ aufgetragen gegen die Spannung) bei $T_C$-30K, monopolaren Pulsen und einem Zellenabstand von 1,3 $\mu$m.

Es resultiert eine Mischung, die ausweislich Fig.1 für den Betrieb von Displays in der "inverse mode" geeignet ist, da der Kurvenverlauf das geforderte Minimum aufweist und die Werte im technisch relevanten Bereich liegen.

**Patentansprüche**

1. Verbindung der Formel (I)

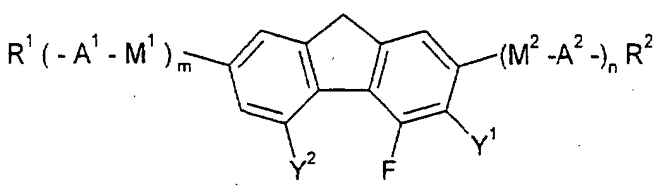

(I)

mit den Bedeutungen:

$Y^1$, $Y^2$ sind gleich oder verschieden H oder F,

$R^1$, $R^2$ sind gleich oder verschieden

Wasserstoff oder ein geradkettiger oder verzweigter Alkylrest mit 1 bis 16 C-Atomen oder ein geradkettiger oder verzweigter Alkenylrest mit 2 bis 16 C-Atomen, worin

a) eine oder mehrere nicht benachbarte und nicht terminale $CH_2$-Gruppen durch -O-, -C(=O)O-, -O-C(=O)-, -O-C(=O)-O-, -C(=O)- oder -$Si(CH_3)_2$- ersetzt sein können, und/oder
b) eine $CH_2$-Gruppe durch -C≡C-, Cyclopropan-1,2-diyl oder Cyclobutan-1,3-diyl ersetzt sein kann, und/oder
c) ein oder mehrere H-Atome durch F oder Cl ersetzt sein können, und/oder
d) die terminale $CH_3$-Gruppe durch eine der folgenden chiralen Gruppen (optisch aktiv oder racemisch) ersetzt sein kann:

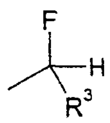
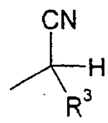
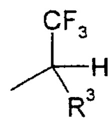
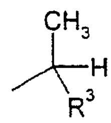

wobei $R^3$ ein geradkettiger oder verzweigter Alkylrest mit 1 bis 12 C-Atomen oder ein geradkettiger oder verzweigter Alkenylrest mit 2 bis 12 C-Atomen ist, worin eine oder mehrere nicht benachbarte und nicht terminale $CH_2$-Gruppen durch -O- ersetzt sein können und/oder ein oder mehrere H-Atome durch F oder Cl ersetzt sein können,

mit der Maßgabe, daß nur einer von $R^1$, $R^2$ Wasserstoff sein kann,

$M^1$, $M^2$ sind gleich oder verschieden
-C(=O)-O-, -O-C(=O)-, $-CH_2-O-$, $-O-CH_2-$, $-CF_2-O-$, $-O-CF_2-$, -CH=CH-, -CF=CF-, -C≡C-, $-CH_2-CH_2-C(=O)-O-$, $-O-C(=O)-CH_2-CH_2-$, $-CH_2-CH_2-$, $-CF_2-CF_2-$, $-(CH_2)_4-$ oder eine Einfachbindung,

$A^1$, $A^2$ sind gleich oder verschieden
1,4-Phenylen, worin ein oder zwei H-Atome durch F, Cl oder CN ersetzt sein können, Pyridin-2,5-diyl, worin ein H-Atom durch F ersetzt sein kann, Pyrimidin-2,5-diyl, 1,4-Cyclohexylen, worin ein oder zwei H-Atome durch $CH_3$ oder F ersetzt sein können, 1-Cyclohexen-1,4-diyl, worin ein H-Atom durch $CH_3$ oder F ersetzt sein kann, oder 1,3-Dioxan-2,5-diyl,

m, n sind unabhängig voneinander 0 oder 1, in Summe jedoch nicht größer als 1.

2. Verbindung nach Anspruch 1, **dadurch gekennzeichnet, daß** in der Formel (I) bedeuten:

$Y^1$ gleich F,

$R^1$, $R^2$ gleich oder verschieden
ein geradkettiger oder verzweigter Alkylrest mit 1 bis 14 C-Atomen oder ein geradkettiger oder verzweigter Alkenylrest mit 2 bis 14 C-Atomen, worin eine oder zwei nicht benachbarte und nicht terminale $CH_2$-Gruppen durch -O- ersetzt sein können und/oder ein oder mehrere H-Atome durch F ersetzt sein können,

$R^3$ ein geradkettiger oder verzweigter Alkylrest mit 1 bis 10 C-Atomen, worin eine nicht terminale $CH_2$-Gruppe durch -O- ersetzt sein kann,

$M^1$, $M^2$ gleich oder verschieden
$-CH_2-O-$, $-O-CH_2-$, $-CH_2-CH_2-$ oder eine Einfachbindung,

$A^1$, $A^2$ gleich oder verschieden
1,4-Phenylen, worin ein oder zwei H-Atome durch F ersetzt sein können, oder 1,4-Cyclohexylen,

m, n unabhängig voneinander 0 oder 1, in Summe jedoch nicht größer als 1.

3. Verbindung nach Anspruch 1, ausgewählt aus der Gruppe der Verbindungen der Formeln (I a) bis (I p)

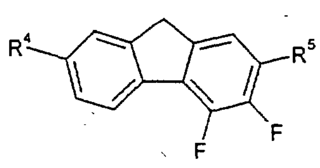

(I a)

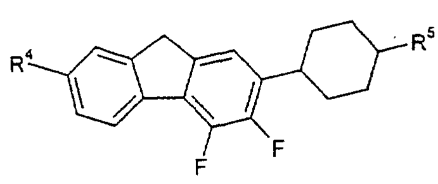

(I b)

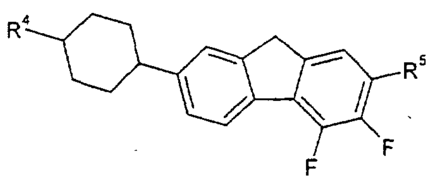

(I c)

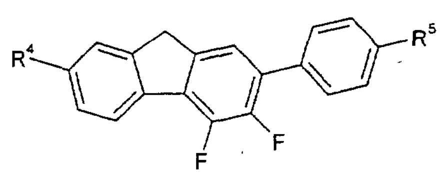

(I d)

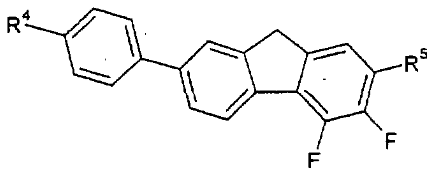

(I e)

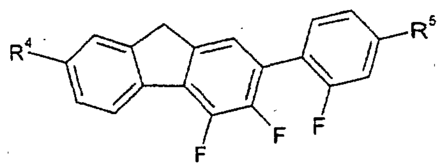

(I f)

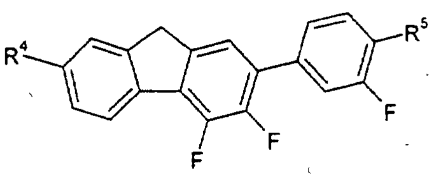

(I g)

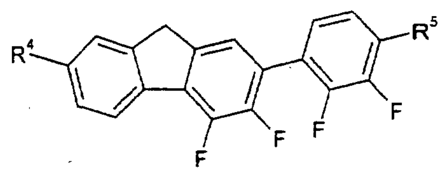

(I h)

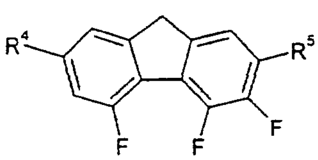

(I i)

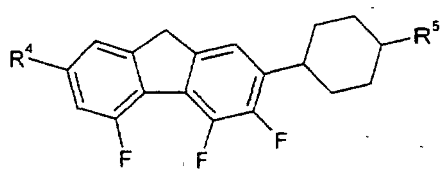

(I k)

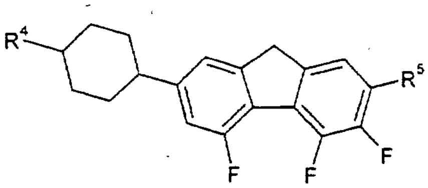

(I l)

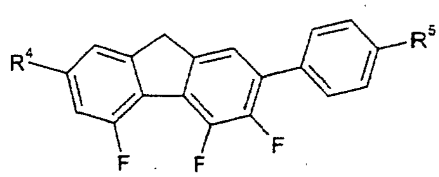

(I m)

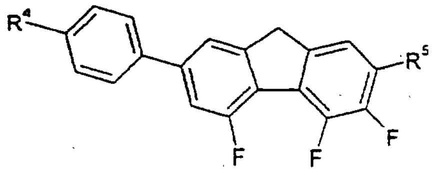

(I n)

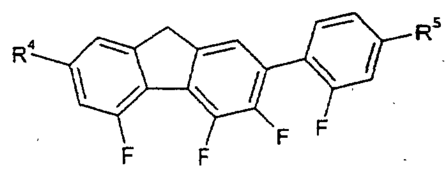

(I o)

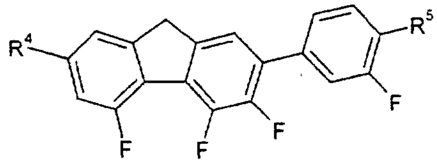

(I p)

mit den Bedeutungen:

$R^4$ und $R^5$ sind gleich oder verschieden ein geradkettiger Alkyl-, Alkenyl-oder Alkoxyrest mit 2 bis 12 C-Atomen.

**4.** Verbindung nach Anspruch 3, ausgewählt aus der Gruppe der Verbindungen der Formeln (I a) bis (I e) und (I i) bis (I n), worin bedeuten:

$R^4$, $R^5$ gleich oder verschieden ein geradkettiger Alkyl-, Alkenyl- oder Alkoxyrest mit 2 bis 10 C-Atomen.

**5.** Verwendung von Verbindungen der Formel (I) gemäß einem der Ansprüche 1 bis 4 in Flüssigkristallmischungen.

**6.** Flüssigkristallmischung, enthaltend eine oder mehrere Verbindungen gemäß einem der Ansprüche 1 bis 4 in einer Menge von 1 bis 40 Gew.-%, bezogen auf die Flüssigkristallmischung.

**7.** Flüssigkristallmischung nach Anspruch 6, **dadurch gekennzeichnet, daß** sie nematisch oder smektisch ist.

**8.** Flüssigkristallmischung nach einem der Ansprüche 6 oder 7, enthaltend neben der/den Verbindung(en) der Formel (I) mindestens drei weitere Komponenten.

**9.** Flüssigkristalldisplay, enthaltend eine Flüssigkristallmischung gemäß einem der Ansprüche 6 bis 8.

**10.** Flüssigkristalldisplay nach Anspruch 9, **dadurch gekennzeichnet, daß** es Aktivmatrix-Elemente enthält oder im Inverse-Mode betrieben wird.

## Claims

**1.** A compound of the formula (I)

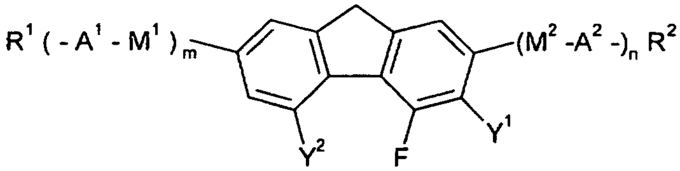

(I)

where
$Y^1$ and $Y^2$ are the same or different and are each H or F,
$R^1$ and $R^2$ are the same or different and are each
hydrogen or a straight-chain or branched alkyl radical having from 1 to 16 carbon atoms or a straight-chain or branched alkenyl radical having from 2 to 16 carbon atoms where

a) one or more nonadjacent and nonterminal $CH_2$ groups may be replaced by -O-, -C(=O)O-, -O-C(=O)-, -O-C(=O)-O-, -C(=O)- or $-Si(CH_3)_2-$, and/or
b) one $CH_2$ group may be replaced by -C≡C-, cyclopropane-1,2-diyl or cyclobutane-1,3-diyl, and/or
c) one or more hydrogen atoms may be replaced by F or Cl, and/or
d) the terminal $CH_3$ group may be replaced by one of the following chiral groups (optically active or racemic):

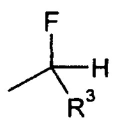 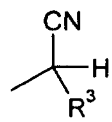 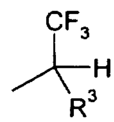 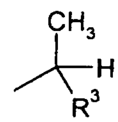

where $R^3$ is a straight-chain or branched alkyl radical having from 1 to 12 carbon atoms or a straight-chain or branched alkenyl radical having from 2 to 12 carbon atoms, in each of which one or more nonadjacent and nonterminal $CH_2$ groups may be replaced by -O- and/or one or more hydrogen atoms may be replaced by F or Cl, with the proviso that only one of $R^1$ and $R^2$ may be hydrogen,
$M^1$ and $M^2$ are the same or different and are each
-C(=O)-O-, -O-C(=O)-, $-CH_2-O-$, $-O-CH_2-$, $-CF_2-O-$, $-O-CF_2-$, -CH=CH-, - CF=CF-, -C≡C-, $-CH_2-CH_2-C(=O)-O-$, $-O-C(=O)-CH_2-CH_2-$, $-CH_2-CH_2-$, $-CF_2-CF_2-$, $-(CH_2)_4-$ or a single bond,
$A^1$ and $A^2$ are the same or different and are each
1,4-phenylene where one or two hydrogen atoms may be replaced by F, Cl or CN, pyridine-2,5-diyl where one hydrogen atom may be replaced by F, pyrimidine-2,5-diyl, 1,4-cyclohexylene where one or two hydrogen atoms may be replaced by $CH_3$ or F, 1-cyclohexene-1,4-diyl where one hydrogen atom may be replaced by $CH_3$ or F, or 1,3-dioxane-2,5-diyl,
m and n are each independently 0 or 1, but in total not greater than 1.

**2.** A compound as claimed in claim 1, wherein, in formula (I),
$Y^1$ is F,
$R^1$ and $R^2$ are the same or different and are each
a straight-chain or branched alkyl radical having from 1 to 14 carbon atoms or a straight-chain or branched alkenyl radical having from 2 to 14 carbon atoms, in each of which one or more nonadjacent and nonterminal $CH_2$ groups may be replaced by -O- and/or one or more hydrogen atoms may be replaced by F,
$R^3$ is a straight-chain or branched alkyl radical having from 1 to 10 carbon atoms where one nonterminal $CH_2$ group may be replaced by -O-,
$M^1$ and $M^2$ are the same or different and are each
$-CH_2-O-$, $-O-CH_2-$, $-CH_2-CH_2-$ or a single bond,
$A^1$ and $A^2$ are the same or different and are each
1,4-phenylene where one or two hydrogen atoms may be replaced by F, or 1,4-cyclohexylene, and
m and n are each independently 0 or 1, but in total not greater than 1.

**3.** A compound as claimed in claim 1, selected from the group of compounds of the formulae (I a) to (I p)

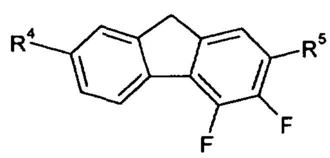

(I a)

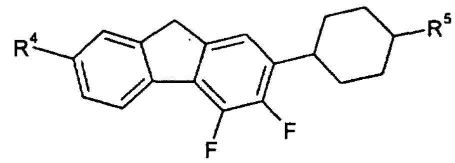

(I b)

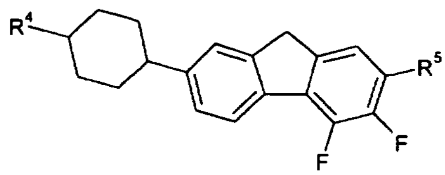

(I c)

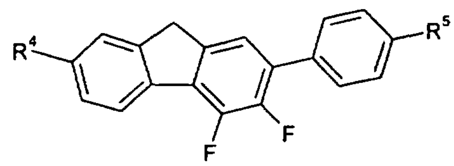

(I d)

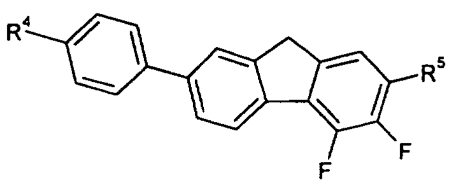

(I e)

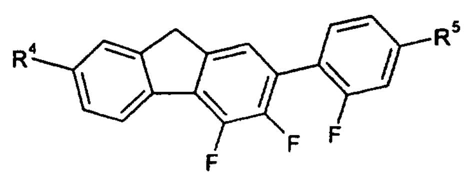

(I f)

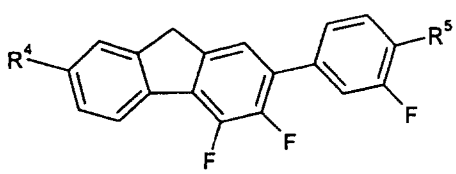

(I g)

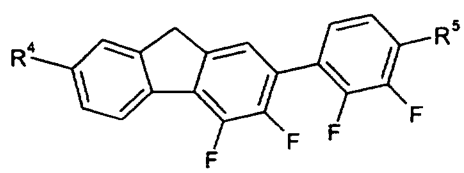

(I h)

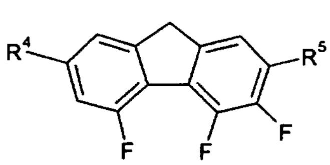

(I i)

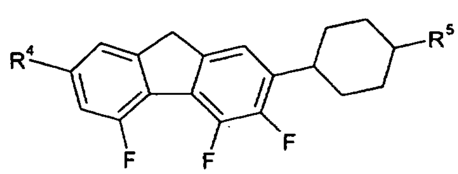

(I k)

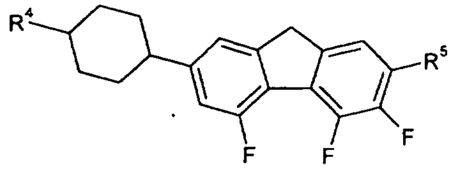

(I l)

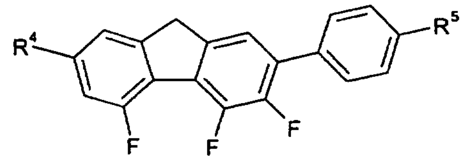

(I m)

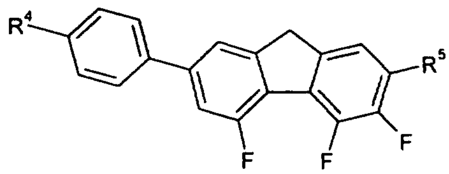

(I n)

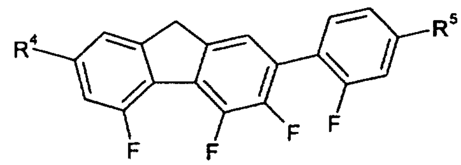

(I o)

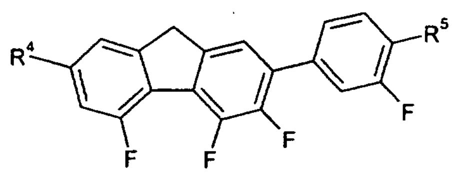

(I p)

where
$R^4$ and $R^5$ are the same or different and are each a straight-chain alkyl, alkenyl or alkoxy radical having from 2 to 12 carbon atoms.

**4.** A compound as claimed in claim 3, selected from the group of compounds of the formulae (I a) to (I e) and (I i) to (I n), where
$R^4$ and $R^5$ are the same or different and are each a straight-chain alkyl, alkenyl or alkoxy radical having from 2 to 10 carbon atoms.

**5.** The use of compounds of the formula (I) as claimed in any of claims 1 to 4 in liquid-crystal mixtures.

**6.** A liquid-crystal mixture comprising one or more compounds as claimed in any of claims 1 to 4 in an amount of from 1 to 40% by weight, based on the liquid-crystal mixture.

**7.** The liquid-crystal mixture as claimed in claim 6, which is nematic or smectic.

**8.** The liquid-crystal mixture as claimed in either of claims 6 or 7, comprising at least three further components in addition to the compound or compounds of the formula (I).

**9.** A liquid-crystal display comprising a liquid-crystal mixture as claimed in any of claims 6 to 8.

**10.** The liquid-crystal display as claimed in claim 9, which comprises active matrix elements or is operated in inverse mode.

## Revendications

**1.** Composé de formule (I)

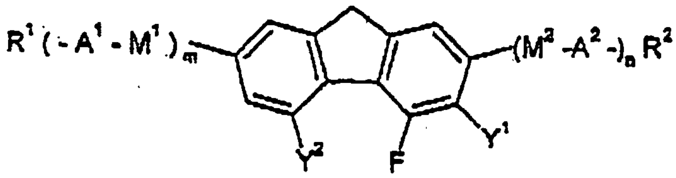

(I)

avec les significations :

$Y^1$, $Y^2$ sont identiques ou différents et représentent un atome d'hydrogène ou de fluor,

$R^1$, $R^2$ sont identiques ou différents et représentent un atome d'hydrogène ou un reste alkyle linéaire ou ramifié présentant 1 à 16 atomes de carbone ou un reste alcényle linéaire ou ramifié présentant 2 à 16 atomes de carbone, où

a) un ou plusieurs groupes $CH_2$ non adjacents et non terminaux peuvent être remplacés par -O-, -C(=O)O-, -O-C(=O)-, -O-C(=O)-O-, -C(=O)- ou $-Si(CH_3)_2-$, et/ou
b) un groupe $CH_2$ peut être remplacé par -C≡C-, cyclopropane-1,2-diyle ou cyclobutane-1,3-diyle, et/ou
c) un ou plusieurs atomes d'hydrogène peuvent être remplacés par un atome de F ou Cl, et/ou
d) le groupe $CH_3$ terminal peut être remplacé par un des groupes chiraux (optiquement actifs ou racémiques) suivants :

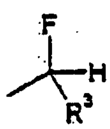
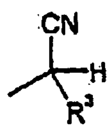
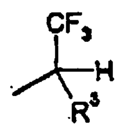
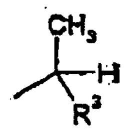

$R^3$ représente un reste alkyle linéaire ou ramifié présentant 1 à 12 atomes de carbone ou un reste alcényle linéaire ou ramifié présentant 2 à 12 atomes de carbone, où un ou plusieurs groupes $CH_2$ non adjacents et non terminaux peuvent être remplacés par -O- et/ou un ou plusieurs atomes d'hydrogène peuvent être remplacés par F ou Cl,
à condition que seulement un de $R^1$, $R^2$ puisse représenter un atome d'hydrogène,

$M^1$, $M^2$ identiques ou différents représentent un groupe -C(=O)O-, -O-C(=O)-, $-CH_2O-$ $-O-CH_2-$, $-CF_2O-$, $-O-CF_2-$, -CH=CH-, -CF=CF-, -C≡C-, $-CH_2-CH_2-C(=O)-O-$, $-O-C(=O)-CH_2-CH_2-$, $-CH_2-CH_2-$, $-CF_2-CF_2-$, $-(CH_2)_4-$ ou une liaison simple,

$A^1$, $A^2$ identiques ou différents représentent un groupe 1,4-phénylène, où un ou deux atomes d'hydrogène peuvent être remplacés par F, Cl ou CN, pyridine-2,5-diyle, où un atome d'hydrogène peut être remplacé par F, pyrimidine-2,5-diyle, 1,4-cyclohexylène, où un ou deux atomes d'hydrogène peuvent être remplacés par $CH_3$ ou F, 1-cyclohexène-1,4-diyle, où un atome d'hydrogène peut être remplacé par un groupe $CH_3$ ou F, ou 1,3-dioxane-2,5-diyle,

m, n indépendamment l'un de l'autre valent 0 ou 1, mais dont la somme n'est pas supérieure à 1.

**2.** Composé selon la revendication 1, **caractérisé en ce que**, à la formule (I)

$Y^1$ représente un atome de F

$R^1$, $R^2$ identiques ou différents représentent un reste alkyle linéaire ou ramifié présentant 1 à 14 atomes de carbone ou un reste alcényle linéaire ou ramifié présentant 2 à 14 atomes de carbone, où un ou deux groupes $CH_2$ non adjacents et non terminaux peuvent être remplacés par -O-et/ou un ou plusieurs atomes d'hydrogène peuvent être remplacés par F,

| | |
|---|---|
| $R^3$ | représente un reste alkyle linéaire ou ramifié présentant 1 à 10 atomes de carbone, où un groupe $CH_2$ non terminal peut être remplacé par un -O-, |
| $M^1$, $M^2$ | identiques ou différents représentent un groupe $-CH_2O-$, $-O-CH_2-$, $-CH_2-CH_2-$ ou une liaison simple, |
| $A^1$, $A^2$ | identiques ou différents représentent un groupe 1,4-phénylène, où un ou deux atomes d'hydrogène peuvent être remplacés par F, ou 1,4-cyclohexylène, |
| m, n | indépendamment l'un de l'autre valent 0 ou 1, mais dont la somme n'est pas supérieure à 1. |

**3.** Composé selon la revendication 1, pris dans le groupe de composés de formules (I a) à (I p)

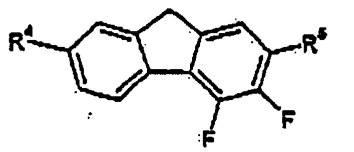

(I a)

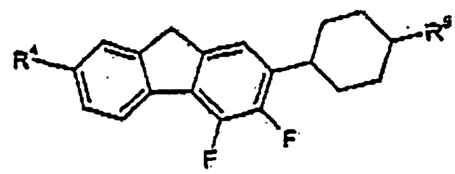

(I b)

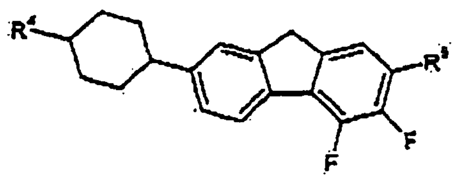

(I c)

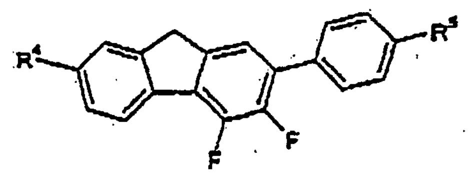

(I d)

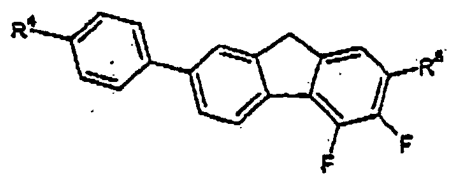

(I e)

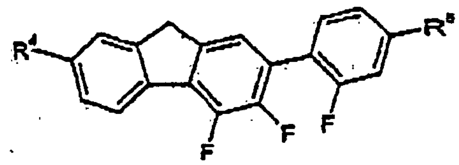

(I f)

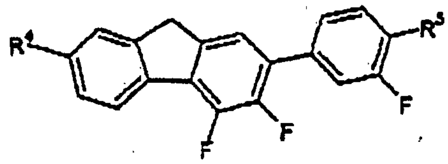

(I g)

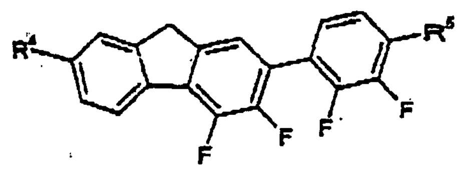

(I h)

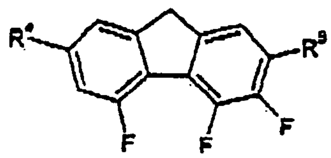

(I i)

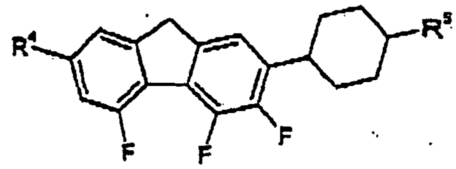

(I k)

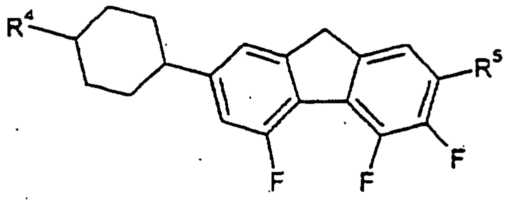

(I l)

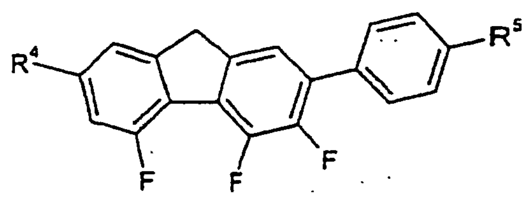

(I m)

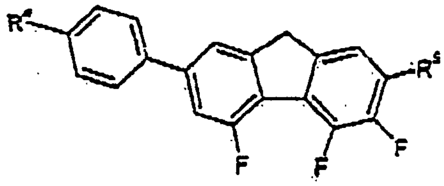

(I n)

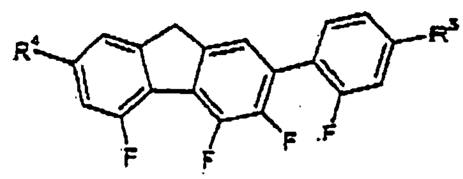

(I o)

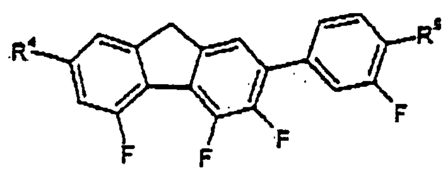

(I p)

avec les significations

$R^4$ et $R^5$ identiques ou différents représentent un reste alkyle, alcényle ou alkoxy linéaire présentant 2 à 12 atomes de carbone.

**4.** Composé selon la revendication 3, pris dans le groupe des composés de formules (I a) à (I e) et (I i) à (I n), où

$R^4$ et $R^5$ identiques ou différents représentent un reste alkyle, alcényle ou alkoxy linéaire présentant 2 à 10 atomes de carbone.

**5.** Utilisation de composés de formule (I) selon l'une des revendications 1 à 4 dans des mélanges de cristaux liquides.

**6.** Mélange de cristaux liquides renfermant un ou plusieurs composés selon l'une des revendications 1 à 4 en une quantité de 1 à 40 % en masse, par rapport au mélange de cristaux liquides.

7. Mélange de cristaux liquides selon la revendication 6, **caractérisé en ce qu'**il est nématique ou smectique.

8. Mélange de cristaux liquides selon l'une des revendications 6 ou 7, renfermant au côté du/des composé(s) de formule (I) au moins trois autres composants.

9. Afficheur à cristal liquide renfermant un mélange de cristaux liquides selon l'une des revendications 6 à 8.

10. Afficheur à cristal liquide selon la revendication 9, **caractérisé en ce qu'**il renferme des éléments de matrice active ou fonctionne en mode inverse.

Fig.1